# EUROPEAN PATENT SPECIFICATION

(11) **EP 4 271 287 B1**
(45) Date of publication and mention of the grant of the patent: **29.01.2025**
(21) Application number: 21841051.2
(22) Date of filing: 02.12.2021
(51) Int. Cl.: A61B 17/221

(54) **DEVICE FOR ELECTRICALLY ENHANCED RETRIEVAL OF MATERIAL FROM VESSEL LUMENS**
GERÄT FÜR ELEKTRISCH VERSTÄRKTE RÜCKGEWINNUNG VON MATERIAL AUS GEFÄSSLUMEN
DISPOSITIF DE RÉCUPÉRATION RENFORCÉE ÉLECTRIQUEMENT D'UNE MATIÈRE PRÉSENTE DANS DES LUMIÈRES DE VAISSEAUX

(30) Priority: 29.12.2020 US 202017247897
(43) Date of publication of application: 08.11.2023
(73) Proprietor: Covidien LP, Mansfield, MA 02048 (US)
(72) Inventor: DAVIDSON, James Andrew, San Juan Capistrano, CA 92675 (US); NGUYEN, Hoai, Westminster, CA 92683 (US)
(74) Representative: Gray, James
(86) International application number: PCT/US2021/061540
(87) International publication number: WO 2022/146613

(56) References cited:
- US-A1- 2002 059 938
- US-A1- 2014 188 127
- US-A1- 2015 133 990
- US-A1- 2019 388 111
- US-A1- 2020 390 458

## Description

### TECHNICAL FIELD

The present technology relates generally to devices and methods for removing obstructions from body lumens. The present invention relates to devices for electrically enhanced removal of clot material from blood vessels, and methods of surface treating such devices.

### BACKGROUND

Many medical procedures use medical device(s) to remove an obstruction (such as clot material) from a body lumen, vessel, or other organ. An inherent risk in such procedures is that mobilizing or otherwise disturbing the obstruction can potentially create further harm if the obstruction or a fragment thereof dislodges from the retrieval device. If all or a portion of the obstruction breaks free from the device and flows downstream, it is highly likely that the free material will become trapped in smaller and more tortuous anatomy. In many cases, the physician will no longer be able to use the same retrieval device to again remove the obstruction because the device may be too large and/or immobile to move the device to the site of the new obstruction.

Procedures for treating ischemic stroke by restoring flow within the cerebral vasculature are subject to the above concerns. The brain relies on its arteries and veins to supply oxygenated blood from the heart and lungs and to remove carbon dioxide and cellular waste from brain tissue. Blockages that interfere with this blood supply eventually cause the brain tissue to stop functioning. If the disruption in blood occurs for a sufficient amount of time, the continued lack of nutrients and oxygen causes irreversible cell death. Accordingly, it is desirable to provide immediate medical treatment of an ischemic stroke.

To access the cerebral vasculature, a physician typically advances a catheter from a remote part of the body (typically a leg) through the abdominal vasculature and into the cerebral region of the vasculature. Once within the cerebral vasculature, the physician deploys a device for retrieval of the obstruction causing the blockage. Concerns about dislodged obstructions or the migration of dislodged fragments increases the duration of the procedure at a time when restoration of blood flow is paramount. Furthermore, a physician might be unaware of one or more fragments that dislodge from the initial obstruction and cause blockage of smaller more distal vessels.

Many physicians currently perform thrombectomies (i.e. clot removal) with stents to resolve ischemic stroke. Typically, the physician deploys a stent into the clot in an attempt to push the clot to the side of the vessel and re-establish blood flow. Tissue plasminogen activator ("tPA") is often injected into the bloodstream through an intravenous line to break down a clot. However, it takes time for the tPA to reach the clot because the tPA must travel through the vasculature and only begins to break up the clot once it reaches the clot material. tPA is also often administered to supplement the effectiveness of the stent. Yet, if attempts at clot dissolution are ineffective or incomplete, the physician can attempt to remove the stent while it is expanded against or enmeshed within the clot. In doing so, the physician must effectively drag the clot through the vasculature, in a proximal direction, into a guide catheter located within vessels in the patient's neck (typically the carotid artery). While this procedure has been shown to be effective in the clinic and easy for the physician to perform, there remain some distinct disadvantages to using this approach.

For example, one disadvantage is that the stent may not sufficiently retain the clot as it pulls the clot to the catheter. In such a case, some or all of the clot might remain in the vasculature. Another risk is that, as the stent mobilizes the clot from the original blockage site, the clot might not adhere to the stent as the stent is withdrawn toward the catheter. This is a particular risk when passing through bifurcations and tortuous anatomy. Furthermore, blood flow can carry the clot (or fragments of the clot) into a branching vessel at a bifurcation. If the clot is successfully brought to the end of the guide catheter in the carotid artery, yet another risk is that the clot may be "stripped" or "sheared" from the stent as the stent enters the guide catheter.

In view of the above, there remains a need for improved devices and methods that can remove occlusions from body lumens and/or vessels.

### SUMMARY

The invention is defined by independent claims 1 and 19. Further embodiments are defined by the dependent claims. No methods of surgery or treatment are claimed. Mechanical thrombectomy (i.e., clot-grabbing and removal) has been effectively used for treatment of ischemic stroke. Although most clots can be retrieved in a single pass attempt, there are instances in which multiple attempts are needed to fully retrieve the clot and restore blood flow through the vessel. Additionally, there exist complications due to detachment of the clot from the interventional element during the retrieval process as the interventional element and clot traverse through tortuous intracranial vascular anatomy. For example, the detached clot or clot fragments can obstruct other arteries leading to secondary strokes. The failure modes that contribute to clot release during retrieval are: (a) boundary conditions at bifurcations; (b) changes in vessel diameter; and (c) vessel tortuosity, amongst others.

Certain blood components, such as platelets and coagulation proteins, display negative electrical charges. The treatment systems of the present technology provide an interventional element and a current generator configured to positively charge the interventional element during one or more stages of a thrombectomy procedure. For example, the current generator may apply a constant or pulsatile direct current (DC) to the interventional element. The positively charged interventional element attracts negatively charged blood components, thereby improving attachment of the thrombus to the interventional element and reducing the number of device passes or attempts necessary to fully retrieve the clot. In some aspects of the present technology, the treatment system includes a core member extending between the current generator and the interventional element. A delivery electrode may be integrated into the core member and/or interventional element, and the treatment system further includes a return electrode that may be disposed at a number of different locations. For example, the return electrode can be a needle, a grounding pad, a conductive element carried by a one or more catheters of the treatment system, a guide wire, and/or any other suitable conductive element configured to complete an electrical circuit with the delivery electrode and the extracorporeally positioned current generator. When the interventional element is placed in the presence of blood (or any other electrolytic medium) and voltage is applied at the terminals of the current generator, current flows along the core member to the interventional element, through the blood, and to the return electrode, thereby positively charging at least a portion of the interventional element and adhering clot material thereto.

One approach to delivering current to an interventional element is to conduct current along a core member coupled to a proximal end of the interventional element. However, the inventors have discovered that this approach can lead to disadvantageous concentration of electrical charge along a proximal portion of the interventional element, with insufficient charge density in more distal portions of the interventional element (e.g., along some or all of the working length of the interventional element). This is particularly true of an interventional element having a proximal portion that tapers to a connection point with the core member. This concentration of current in the proximal portion can reduce the efficacy of electrostatic enhancement of clot adhesion, as the mechanical clot engagement occurs primarily at a location distal to the region at which the charge density is greatest. Additionally, when used in an aqueous chloride environment such as the blood, hydrogen and chlorine gas bubbles can form along the surface of the interventional element in areas with high surface charge density (e.g., along a proximal portion of the interventional element).

The treatment systems and methods of the present technology can overcome these and other problems by varying features of the interventional element to achieve the desired electrical properties. For example, in some embodiments, some or all of the interventional element can be surface treated, coated, or otherwise modified to alter its electrical properties. The electrical properties of the interventional element can be varied spatially across different regions of the interventional element so as to improve the electrical charge distribution over its surface during use in the body. In some embodiments, a proximal region of the interventional element can be at least partially electrically insulated such that the distal region is more electrically conductive than the proximal region. This variation in conductivity can help achieve a more desirable charge distribution across the interventional element, and can avoid the undesirable concentration of electrical charge at a proximal region as described above. In various embodiments, some or all of the interventional element can be electrically insulated by surface treating, coating, or otherwise modifying the interventional element to reduce its electrical conductivity.

According to some aspects of the present technology, electrochemical anodization can be utilized to alter the electrical properties of the interventional element. For example, anodization can be used to increase a thickness of the naturally occurring oxide layer disposed over the interventional element. As the oxide layer thickness increases, the surface conductivity of the interventional element decreases, thereby at least partially electrically insulating that portion of the interventional element. In some embodiments, anodization can be used to achieve varying thicknesses of an oxide layer over the surface of the interventional element, such as by having a thicker oxide layer in a proximal region and a thinner oxide layer in a distal region. By tuning the thickness of the oxide layer over the interventional element, a favorable electrical charge distribution can be achieved (e.g., by achieving a more uniform charge distribution and/or by concentrating charge distribution into distal regions or along the working length of the interventional element).

Additionally or alternatively to insulating a portion of the interventional element, the distal region (or any other suitable portion of the interventional element) can be coated, surface treated, or otherwise modified to increase its conductivity. For example, a distal region can be coated with gold or other highly conductive materials so as to increase the electrical conductivity of the distal region.

Additional features and advantages of the present technology are described below, and in part will be apparent from the description, or may be learned by practice of the present technology. The advantages of the present technology will be realized and attained by the structure particularly pointed out in the written description and claims hereof as well as the drawings.

Background art: US2020/390458 and US2019/388111 disclose a thrombectomy system US2002/059938 discloses a tissue localizing device.

Additional features and advantages of the present technology are described below, and in part will be apparent from the description, or may be learned by practice of the present technology. The advantages of the present technology will be realized and attained by the structure particularly pointed out in the written description and claims hereof as well as the appended drawings.

### BRIEF DESCRIPTION OF THE DRAWINGS

Many aspects of the present technology can be better understood with reference to the following drawings. The components in the drawings are not necessarily to scale. Instead, emphasis is placed on illustrating clearly the principles of the present disclosure.
Figure 1A shows a perspective view of an electrically enhanced treatment system for retrieving material from a body lumen, in accordance with one or more embodiments of the present technology.
Figures 1B and 1C are schematic views of different embodiments of the current generator illustrated in Figure 1A.
Figure 2A is a side schematic view of a portion of the treatment system of Figure 1A.
Figures 2B is a side schematic cross-sectional view of a portion of the treatment system shown in Figure 2A.
Figure 3A illustrates an interventional element with one or more coatings in accordance with embodiments of the present technology.
Figure 3B illustrates an interventional element with an overlying oxide layer in accordance with embodiments of the present technology.
Figure 4 is a plan view of an interventional element in accordance with embodiments of the present technology.
Figure 5 illustrates anodization of an interventional element in accordance with embodiments of the present technology.
Figures 6-8 illustrate additional embodiments of interventional elements.
Figures 9A-9G illustrate a method of removing clot material from a blood vessel lumen using an electrically enhanced treatment system.
Figures 10A-10E illustrate sample waveforms for electrically enhanced removal of material from vessel lumens in accordance with one or more embodiments of the present disclosure.

### DETAILED DESCRIPTION

The present technology provides devices, systems, and methods for removing clot material from a blood vessel lumen. Although many of the embodiments are described below with respect to devices, systems, and methods for treating a cerebral or intracranial embolism, other applications and other embodiments in addition to those described herein are within the scope of the technology. For example, the treatment systems and methods of the present technology may be used to remove emboli from corporeal lumens other than blood vessels (e.g., the digestive tract, etc.) and/or may be used to remove emboli from blood vessels outside of the brain (e.g., pulmonary, abdominal, cervical, or thoracic blood vessels, or peripheral blood vessels including those within the legs or arms, etc.). In addition, the treatment systems and methods of the present technology may be used to remove luminal obstructions other than clot material (e.g., plaque, resected tissue, foreign material, etc.).

### I. Select Embodiments of Electrically Enhanced Treatment Systems

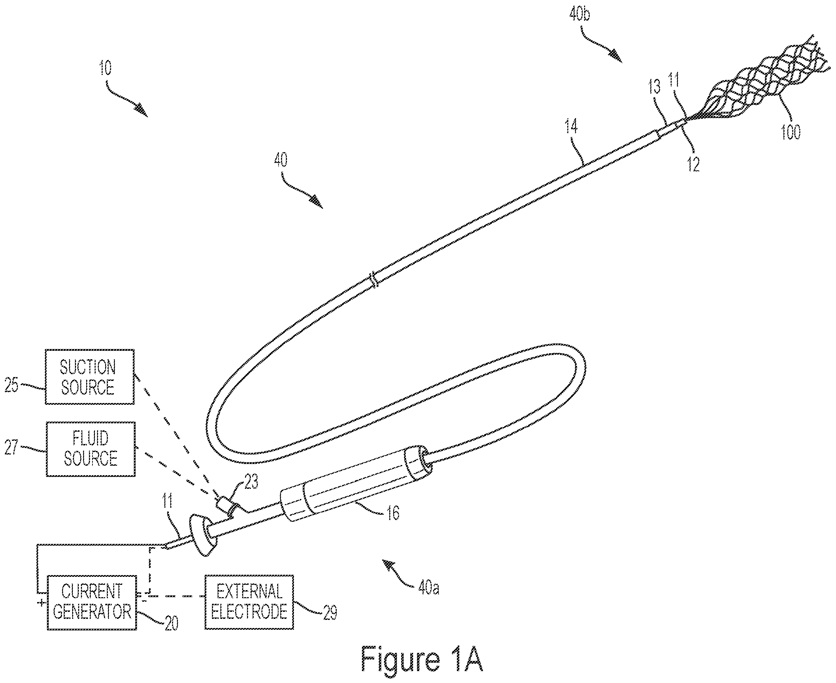

Figure 1A illustrates a view of an electrically enhanced treatment system 10 according to one or more embodiments of the present technology. As shown in Figure 1A, the treatment system 10 can include a current generator 20 and a treatment device 40 having a proximal portion 40a configured to be coupled to the current generator 20 and a distal portion 40b configured to be intravascularly positioned within a blood vessel (such as an intracranial blood vessel) at a treatment site at or proximate a thrombus. The treatment device 40 includes an interventional element 100 at the distal portion 10b, a handle 16 at the proximal portion 10a, and a plurality of elongated shafts or members extending therebetween. For example, in some embodiments, such as that shown in Figure 1A, the treatment device 40 includes a first catheter 14 (such as a balloon guide catheter), a second catheter 13 (such as a distal access catheter or aspiration catheter) configured to be slidably disposed within a lumen of the first catheter 14, a third catheter 12 (such as a microcatheter) configured to be slidably disposed within a lumen of the second catheter 13, and a core member 11 configured to be slidably disposed within a lumen of the third catheter 12. In some embodiments, the treatment device 40 does not include the second catheter 13. The first catheter 14 can be coupled to the handle 16, which provides proximal access to the core member 11 that engages the interventional element 100 at a distal end thereof. The current generator 20 may be coupled to a proximal portion of one or more of the core member 11, the third catheter 12, the second catheter 13, and/or the first catheter 14 to provide an electrically charged environment at the distal portion 40b of the treatment device 40, as described in more detail below.

In some embodiments, the treatment system 10 includes a suction source 25 (e.g., a syringe, a pump, etc.) configured to be fluidly coupled (e.g., via a connector 23) to a proximal portion of one or more of the first catheter 14, the second catheter 13, and/or the third catheter 12 to apply negative pressure therethrough. In some embodiments, the treatment system 10 includes a fluid source 27 (e.g., a fluid reservoir, a syringe, pump, etc.) configured to be fluidly coupled (e.g., via the connector 23) to a proximal portion of one or more of the first catheter 14, the second catheter 13, and/or the third catheter 12 to supply fluid (e.g., saline, contrast agents, a drug such as a thrombolytic agent, etc.) to the treatment site.

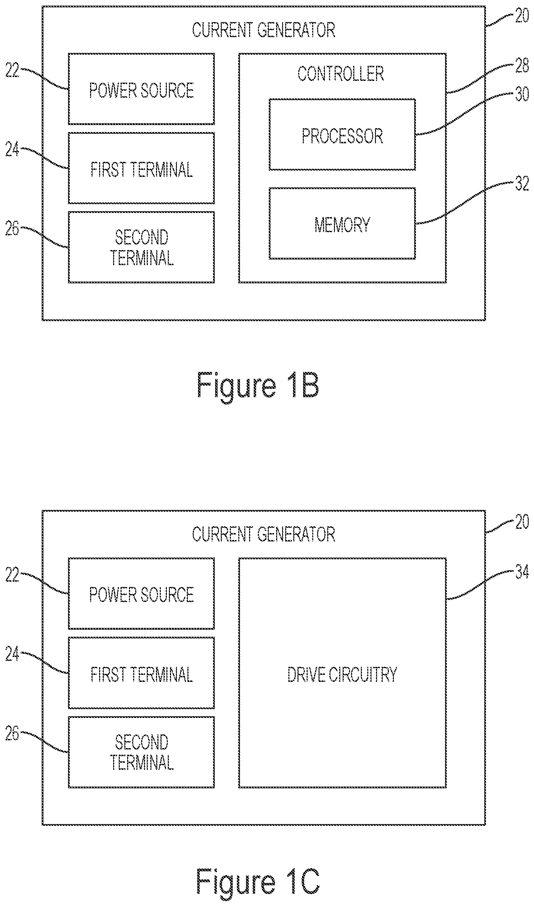

According to some embodiments, the current generator 20 can include an electrical generator configured to output medically useful electric current. Figures 1B and 1C are schematic views of different embodiments of the current generator 20. With reference to Figure 1B, the current generator 20 can include a power source 22, a first terminal 24, a second terminal 26, and a controller 28. The controller 28 includes a processor 30 coupled to a memory 32 that stores instructions (e.g., in the form of software, code or program instructions executable by the processor or controller) for causing the power source 22 to deliver electric current according to certain parameters provided by the software, code, etc. The power source 22 of the current generator 20 may include a direct current power supply, an alternating current power supply, and/or a power supply switchable between a direct current and an alternating current. The current generator 20 can include a suitable controller that can be used to control various parameters of the energy output by the power source or generator, such as intensity, amplitude, duration, frequency, duty cycle, and polarity. For example, the current generator 20 can provide a voltage of about 2 volts to about 28 volts and a current of about 0.5 mA to about 20 mA.

Figure 1C illustrates another embodiment of the current generator 20, in which the controller 28 of Figure 1B is replaced with drive circuitry 34. In this embodiment, the current generator 20 can include hardwired circuit elements to provide the desired waveform delivery rather than a software-based generator of Figure 1B. The drive circuitry 34 can include, for example, analog circuit elements (e.g., resistors, diodes, switches, etc.) that are configured to cause the power source 22 to deliver electric current via the first and second terminals 24, 26 according to the desired parameters. For example, the drive circuitry 34 can be configured to cause the power source 22 to deliver periodic waveforms via the first and second terminals 24, 26.

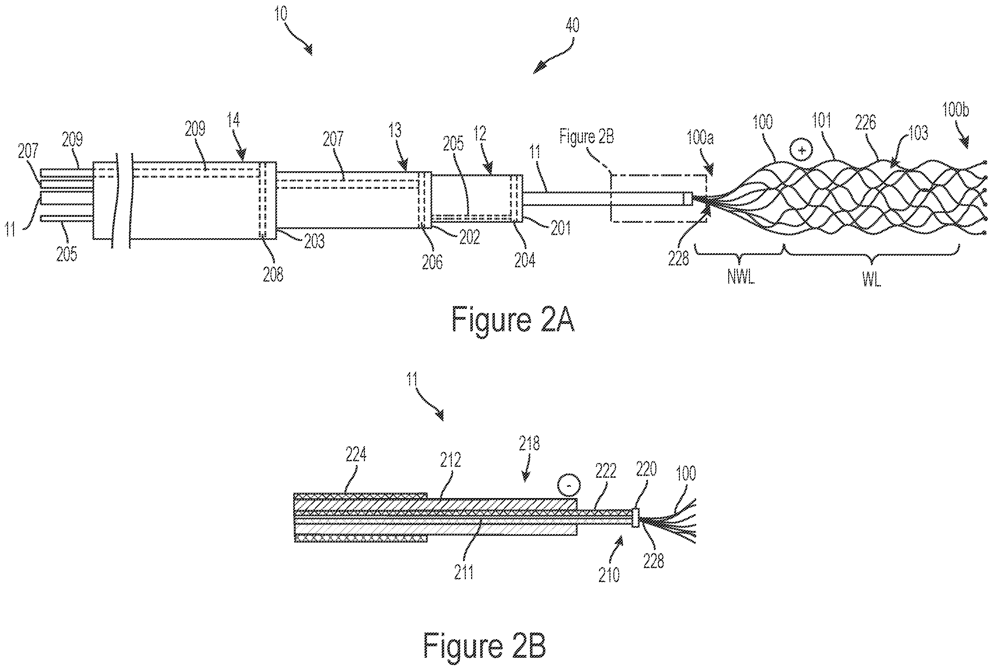

As noted above, the current generator 20 may be coupled to a proximal portion of the core member 11, and/or a proximal portion of the third catheter 12, the second catheter 13, and/or first catheter 14 to provide an electric current to the interventional element 100. For example, in some embodiments, both terminals of the current generator 20 are coupled to the core member 11 such that the core member 11 functions as both a delivery electrode or conductive path (i.e., transmitting current from the current generator 20 to the treatment site) and a return electrode or conductive path (i.e., transmitting current from the treatment site to the current generator 20) (described in greater detail below with reference to Figure 2B). In other embodiments, the return electrode can be separate from the core member 11. For example, the return electrode can be carried by one or more of the third catheter 12, the second catheter 13, and/or first catheter 14. In some embodiments, the return electrode can be provided via one or more external electrodes 29 (Figure 1A), such as a needle puncturing the patient or a grounding pad applied to the patient's skin. In some embodiments, the return electrode can be an insulated guide wire having an exposed, electrically conductive portion at its distal end.

Figure 2A is a side schematic view of a portion of the treatment device 40 shown in Figure 1A. The system 10 can include multiple (e.g., two or more), distinct conductive paths or channels for passing electrical current along the system 10. The interventional element 100 can serve as one electrode (e.g., the delivery electrode) in electrical communication with a conductive path integrated into the core member 11. Another of the conductive paths of the system 10 can be in electrical communication with another electrode (e.g., a return electrode). The various embodiments of the core member 11 can be sized for insertion into a bodily lumen, such as a blood vessel, and can be configured to push and pull a device such as the interventional element 100 along the bodily lumen.

As noted above, the interventional element 100 can serve as the delivery electrode and be electrically coupled to a positive terminal of the current generator 20 (Figure 1A). As shown in Figure 2B, in some embodiments, the core member 11 can include an elongate conductive shaft 211 (e.g., a pushwire) extending along the length of the core member 11. The shaft can be in electrical communication with the current generator 20 (Figure 1A) at its proximal end and the interventional element 100 at its distal end. The shaft can be insulated along at least a portion of its length, with exposed portions permitting electrical communication with the current generator 20 and the interventional element 100.

The return electrode(s) can assume a variety of configurations in different embodiments. For example, in some embodiments, the return electrode is an external electrode 29 (Figure 1A), such as a needle or grounding pad that is applied to a patient's skin. The needle or grounding pad can be coupled via one or more leads to the current generator 20 to complete the electrical circuit. In some embodiments, the return electrode is carried by a surrounding catheter (e.g., third catheter 12, second catheter 13, and/or first catheter 14), as described in more detail below.

According to some embodiments, for example as shown in FIG. 2A, the catheters 12, 13, and 14 can each be formed as a generally tubular member extending along and about a central axis and terminating in a respective distal end 201, 202, and 203. According to some embodiments, the third catheter 12 is generally constructed to track over a conventional guidewire in the cervical anatomy and into the cerebral vessels associated with the brain and may also be chosen according to several standard designs that are generally available. Accordingly, the third catheter 12 can have a length that is at least 125 cm long, and more particularly may be between about 125 cm and about 175 cm long. Other designs and dimensions are contemplated.

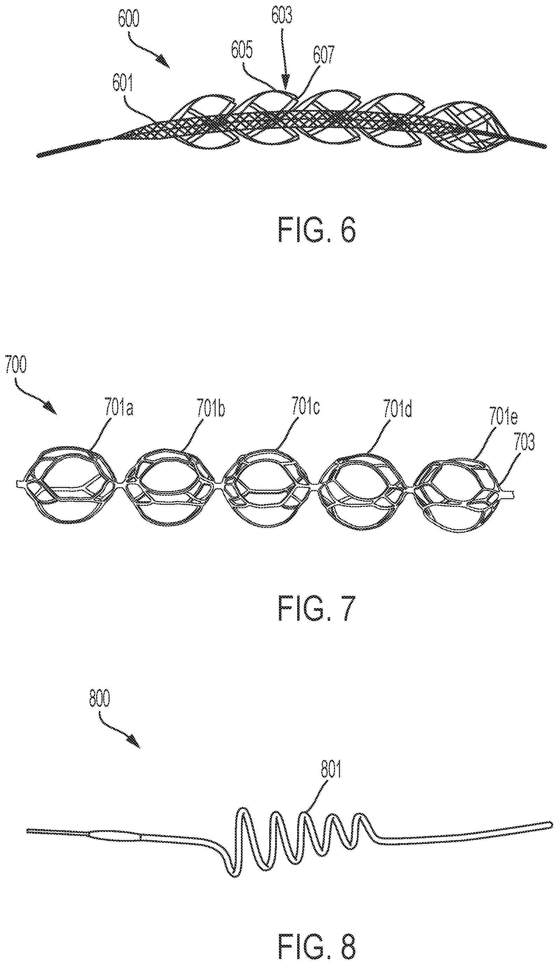

The second catheter 13 can be sized and configured to be slidably receive the third catheter 12 therethrough. As noted above, the second catheter 13 can be coupled at a proximal portion to a suction source 25 (Figure 1A) such as a pump or syringe in order to supply negative pressure to a treatment site. The first catheter 14 can be sized and configured to slidably receive both the second catheter 13 and the third catheter 12 therethrough. In some embodiments, the first catheter 14 is a balloon-guide catheter having an inflatable balloon or other expandable member that can be used to anchor the first catheter 14 with respect to a surrounding vessel. As described in more detail below with respect to Figures 6A-6G, in operation the first catheter 14 can first be advanced through a vessel and then a balloon can be expanded to anchor the first catheter 14 in place and/or arrest blood flow from areas proximal of the balloon. Next, the second catheter 13 can be advanced through the first catheter 14 until its distal end 202 extends distally beyond the distal end 203 of the first catheter 14. The second catheter 13 can be positioned such that its distal end 202 is adjacent a treatment site (e.g., a site of a blood clot within the vessel). The third catheter 12 may then be advanced through the second catheter 13 until its distal end 201 extends distally beyond the distal end 202 of the second catheter 13. The interventional element 100 may then be advanced through the third catheter 12 for delivery to the treatment site.

According to some embodiments, the bodies of the catheters 12, 13, and 14 can be made from various thermoplastics, e.g., polytetrafluoroethylene (PTFE or TEFLON^{®}), fluorinated ethylene propylene (FEP), high-density polyethylene (HDPE), polyether ether ketone (PEEK), etc., which can optionally be lined on the inner surface of the catheters or an adjacent surface with a hydrophilic material such as polyvinylpyrrolidone (PVP) or some other plastic coating. Additionally, either surface can be coated with various combinations of different materials, depending upon the desired results.

According to some embodiments, an electrode 204 is provided at a distal end region of the third catheter 12. The electrode 204 can form an annular ring that extends entirely circumferentially about the central axis of the third catheter 12. Alternatively or in combination, the electrode 204 can extend less than entirely circumferentially around the third catheter 12. For example, the electrode 204 may be entirely disposed on one radial side of the central axis. By further example, the electrode 204 may provide a plurality of discrete, noncontiguous electrode sections about the central axis. Such sections of the electrode 204 can be in electrical communication with a common conductive path so as to function collectively as a single electrode, or with multiple separate such paths to allow the sections to function independently if desired. The electrode 201 can be a band, a wire, or a coil embedded in the wall of the third catheter 12. According to some embodiments, the electrode 204 can be longitudinally separated from the distal end 201 of the third catheter 12 by a non-conductive portion of the third catheter 12. Alternatively, a distal portion of the electrode 204 can extend to the distal end 201 of the third catheter 12, such that the electrode 204 forms a portion of the distal end 201. According to some embodiments, an inner surface of the electrode 204 can be flush with an inner surface of the third catheter 12. Alternatively or in combination, the inner surface of the electrode 204 can extend more radially inwardly relative to the inner surface of the third catheter 12 (e.g., providing a "step"). Alternatively or in combination, the inner surface of the electrode 204 can extend less radially inwardly relative to the inner surface of the third catheter 12 (e.g., be recessed into the body). According to some embodiments, the electrode 204 can be surrounded radially by an outer section of the third catheter 12 to provide insulation from an external environment. In some embodiments, an outer surface of the electrode 204 can be flush with an outer surface of the third catheter 12 and can provide an exposed, radially outwardly facing electrode surface. In such instances, a radially inner section of the third catheter 12 can provide insulation from the environment within the lumen of the third catheter 12.

The electrode 204 can include one or more rings, one or more coils or other suitable conductive structures, and can each form at least one surface (e.g., an inner surface or an outer surface) that is exposed and configured for electrical activity or conduction. The electrode 204 can have a fixed inner diameter or size, or a radially expandable inner diameter or size. In some embodiments, the electrode 204 is a "painted" electrode. The electrode can include platinum, platinum alloys (e.g., 92% platinum and 8% tungsten, 90% platinum and 10% iridium), gold, cobalt-chromium, stainless steel (e.g., 304 or 316), nitinol, and combinations thereof, or any suitable conductive materials, metals or alloys.

In some embodiments, the electrode 204 can be a separate expandable member coupled to an outer surface of the third catheter 12, for example a braid, stent, or other conductive element coupled to an outer surface of the distal portion of the third catheter 12. In some embodiments, the electrode can be part of a flow-arrest element such as an expandable braid coupled to an occlusion balloon.

According to some embodiments, the electrode 204 can be electrically connected to the current generator 20 via a conductive lead 205. The conductive lead 205 can extend proximally along or within the wall of the third catheter 12 to or beyond the proximal end of the third catheter 12. The conductive lead 205 can include more than one conductive path extending within the walls of the third catheter 12. According to some embodiments, the conductive lead 205 can form a helical coil along or within at least a portion of the third catheter 12. Alternatively or in combination, the conductive lead 205 can form a braided, woven, or lattice structure along or within at least a portion of the third catheter 12. In some embodiments, the conductive lead 205 can be a conductive element (e.g., a wire, coil, etc.) wrapped around an external surface of the third catheter 12. In such instances, the conductive lead 205 can be coated with an insulative material along at least a portion of its length. The insulative material can be, for example, Parylene, PTFE, or other suitable insulative material.

In some embodiments, the second catheter 13 and/or the first catheter 14 can be similarly equipped with corresponding electrodes instead of or in addition to the third catheter 12 or the core member 11. For example, the second catheter 13 may include an electrode 206 disposed at a distal end region of the second catheter 13. The electrode 206 can be electrically connected to the current generator 20 (Figure 1A) via a conductive lead 207 which extends proximally along the second catheter 13. The configuration of the electrode 206 and the corresponding conductive lead 207 can be similar to any of the variations described above with respect to the electrode 204 and the conductive lead 205 of the third catheter 12.

In some embodiments, the first catheter 14 includes an electrode 208 disposed at a distal end region of the first catheter 14. The electrode 208 can be electrically connected to the current generator 20 (Figure 1A) via a conductive lead 209 which extends proximally along the first catheter 14. The configuration of the electrode 208 and the corresponding conductive lead 209 can be similar to any of the variations described above with respect to the electrode 204 and the conductive lead 205 of the third catheter 12.

In various embodiments, the system can include any combination of the electrodes 204, 206, and 208 described above. For example, the system may include the electrode 204 and the corresponding conductive lead 205 of the third catheter 12, while the second catheter 13 and the first catheter 14 may be provided with no electrodes or conductive leads therein. In other embodiments, the system may only include the electrode 206 of the second catheter 13, while the third catheter 12 and the first catheter 14 may be provided with no electrodes or conductive leads therein. In still other embodiments, the system may include only the electrode 208 of the first catheter 14, while the third catheter 12 and the second catheter 13 are provided with no electrodes or corresponding conductive leads therein. In some embodiments, any two of the catheters 12, 13, or 14 can be provided with electrodes and corresponding leads, while the remaining catheter may have no electrode or conductive lead therein.

In the configuration illustrated in Figure 2A, one or more of electrodes 204, 206, or 208 can be coupled to a negative terminal of the current generator 20, while the interventional element 100 can be coupled to the positive terminal of the current generator 20 via the core member 11. As a result, when voltage is applied at the terminals and the interventional element 100 placed in the presence of blood (or any other electrolytic medium), current flows from the interventional element 100, through the blood or medium, and to the return electrode. The return electrode may a conductive element carried by one or more of the catheters 12, 13, or 14 as described above, or the core member 11, or in some embodiments the return electrode can be an external electrode 29 (Figure 1A) such as needle or grounding pad.

In some embodiments, one or more catheters carrying an electrode can be used without an electrically coupled interventional element 100. In various embodiments, the interventional element 100 may be omitted altogether (as in Figures 6A-6B described below), or the interventional element 100 may be included but may not be electrically coupled to the current generator 20. In such cases, a catheter-based electrode (e.g., the electrode 204 carried by the third catheter 12, the electrode 206 carried by the second catheter 13, or the electrode 208 carried by the first catheter 14) can function as the delivery electrode, and a separate return electrode can be provided either in the form of another catheter-based electrode (either carried by the same catheter or carried by another catheter) or as an external electrode (e.g., a needle or grounding pad). In instances in which a single catheter carries two electrodes, one electrode may be provided on an exterior surface of the catheter while the other electrode may be provided on an inner surface of the catheter. For example, the second catheter 13 may include a delivery electrode in the form of a conductive band disposed on an inner surface of the catheter 13, in addition to a return electrode in the form of a conductive band disposed on an outer surface of the catheter 13.

As described in more detail in Figure 2B, in some embodiments the return electrode can be integrated into the core member 11 of the treatment system 10, such that the core member 11 carries two separate conductive paths along its length. Figures 2B is a side schematic cross-sectional view of a portion of the treatment system shown in Figure 2A, in accordance with some embodiments. As shown in Figure 2B, the core member 11 includes an elongate conductive shaft 211 and an elongate tubular member 212 having a lumen through which the shaft 211 extends. The shaft 211 has a distal portion 210, and the tubular member 212 has a distal portion 218. Both the shaft 211 and the tubular member 212 are electrically conductive along their respective lengths. In some embodiments, the positions of the shaft 211 and the tubular member 212 are fixed relative to one another. For example, in some embodiments the shaft 211 is not slidable or rotatable with respect to the tubular member 212 such that the core member 11 can be pushed or pulled without relative movement between the shaft 211 and the tubular member 212 and/or other individual components of the core member 11.

In some embodiments, the shaft 211 can be a solid pushwire, for example a wire made of Nitinol, stainless steel, or other metal or alloy. The shaft 211 may be thinner than would otherwise be required due to the additional structural column strength provided by the surrounding tubular member 212. The tubular member 212 can be a hollow wire, hypotube, braid, coil, or other suitable member(s), or a combination of wire(s), tube(s), braid(s), coil(s), etc. In some embodiments, the tubular member 212 can be a laser-cut hypotube having a spiral cut pattern (or other pattern of cut voids) formed in its sidewall along at least a portion of its length. The tubular member 212 can be made of stainless steel (e.g., 304 SS), Nitinol, and/or other alloy. In at least some embodiments, the tubular member 212 can have a laser cut pattern to achieve the desired mechanical characteristics (e.g., column strength, flexibility, kink-resistance, etc.).

The core member 11 can also include an adhesive or a mechanical coupler such as a crimped band or marker band 220 disposed at the distal end of the core member 11, and the marker band 220 can optionally couple the distal end of the core member 11 to the interventional element 100. The marker band 220 can be radiopaque, for example including platinum or other radiopaque material, thereby enabling visualization of the proximal end of the interventional element 100 under fluoroscopy. In some embodiments, additional radiopaque markers can be disposed at various locations along the treatment system 10, for example along the shaft 211, the tubular member 212, or the interventional element 100 (e.g., at the distal end, or along the length, of the interventional element 100).

In at least some embodiments, the core member 11 also includes a first insulating layer or material 222 extending between the shaft 211 and the surrounding tubular member 212. The first insulating material 222 can be, for example, PTFE (polytetrafluoroethylene or TEFLON^{™}) or any other suitable electrically insulating coating (e.g., polyimide, oxide, ETFE-based coatings, or any suitable dielectric polymer). In some embodiments, the first insulating material 222 extends along substantially the entire length of the shaft 211. In some embodiments, the first insulating material 222 separates and electrically insulates the shaft 211 and the tubular member 212 along the entire length of the tubular member 212. In some embodiments, the first insulating material 222 does not cover the proximal-most portion of the shaft 211, providing an exposed region of the shaft to which the current generator 20 (Figure 1A) can be electrically coupled. In some embodiments, for example, the first insulating material 222 terminates proximally at the proximal terminus of the shaft, and the current generator 20 (Figure 1A) can electrically couple to the shaft 211 at its proximal terminus, for example using a coaxial connector.

The core member 11 can additionally include a second insulating layer or material 224 surrounding the tubular member 212 along at least a portion of its length. The second insulating layer 224 can be, for example, PTFE or any other suitable electrically insulative coating (e.g., polyimide, oxide, ETFE based coatings or any suitable dielectric polymer). In some embodiments, the distal portion 218 of the tubular member 212 is not covered by the second insulating layer 224, leaving an exposed conductive surface at the distal portion 218. In some embodiments, the length of the exposed distal portion 218 of the tubular member 212 can be at least (or equal to) 1, 2, 3, 4, 5, 6, or more inches (To be considered for the whole description: 1 inch ≈ 25,4 mm). In some embodiments, the length of the exposed distal portion 218 of the tubular member 212 can be between at least 1 and 10 inches, or between 2 inches and 8 inches, or between 3 and 7 inches, or between 4 and 6 inches, or about 5 inches. This exposed portion of the distal portion 218 of the tubular member 212 provides a return path for current supplied to the delivery electrode (e.g. the entirety or a portion of the interventional element 100), as described in more detail below. In some embodiments, the second insulating material 224 does not cover the proximal-most portion of the tubular member 212, providing an exposed region of the tubular member 212 to which the current generator 20 (Figure 1A) can be electrically coupled. In some embodiments, the second insulating material 224 proximally terminates at the proximal terminus of the tubular member 212, and the current generator 20 can electrically couple to the tubular member 212 at its proximal terminus, for example using a coaxial connector.

The core member 11 can also include a retraction marker in the proximal portion of the tubular member 212. The retraction marker can be a visible indicator to guide a clinician when proximally retracting an overlying catheter with respect to the core member 11. For example, the retraction marker can be positioned such that when a proximal end of the overlying catheter is retracted to be positioned at or near the retraction marker, the distal portion 218 of the tubular member 212 is positioned distally beyond a distal end of the catheter. In this position, the exposed distal portion 218 of the tubular member 212 is exposed to the surrounding environment (e.g., blood, tissue, etc.), and can serve as a return electrode for the core member 11.

The proximal end of the shaft 211 can be electrically coupled to the positive terminal of the current generator 20, and the proximal end of the tubular member 212 can be electrically coupled to the negative terminal of the current generator 20. During operation, the treatment system 10 provides an electrical circuit in which current flows from the positive terminal of the current generator 20, distally through the shaft 211, the interventional element 100, and the surrounding media (e.g., blood, tissue, thrombus, etc.) before returning back to the exposed distal portion 218 of the tubular member, proximally through the tubular member 212, and back to the negative terminal of the current generator 20 (Figure 1A).

As noted above, the current generator 20 (Figure 1A) can include a power source and either a processor coupled to a memory that stores instructions for causing the power source to deliver electric current according to certain parameters, or hardwired circuit elements configured to deliver electric current according to the desired parameters. The current generator 20 may be integrated into the core member 11 or may be removably coupled to the core member 11, for example via clips, wires, plugs or other suitable connectors. Particular parameters of the energy provided by the current generator 20 are described in more detail below with respect to Figures 7A-7E.

In certain embodiments, the polarities of the current generator 20 can be switched, so that the negative terminal is electrically coupled to the shaft 211 and the positive terminal is electrically coupled to the tubular member 212. This can be advantageous when, for example, attempting to attract predominantly positively charged material to the interventional element 100, or when attempting to break up a clot rather than grasp it with an interventional element. In some embodiments alternating current (AC) signals may be used rather than DC. In certain instances, AC signals may advantageously help break apart a thrombus or other material.

### II. Select Embodiments of Interventional Elements

Referring still to Figures 2A and 2B, in some embodiments the interventional element 100 can be a metallic or electrically conductive thrombectomy device. The interventional element 100 can have a low-profile, constrained or compressed configuration (not shown) for intravascular delivery to the treatment site within the third catheter 12, and an expanded configuration for securing and/or engaging clot material and/or for restoring blood flow at the treatment site. The interventional element 100 has a proximal end portion 100a that may be coupled to the core member 11 and a distal end portion 100b. The interventional element 100 further includes an open cell framework or body 226 and a coupling region 228 extending proximally from the body 226. In some embodiments, the body 226 of the interventional element 100 can be generally tubular (e.g., cylindrical), and the proximal end portion 100a of the interventional element 100 can taper proximally to the coupling region 228.

In various embodiments, the interventional element 100 can take any number of forms, for example a removal device, a thrombectomy device, or other suitable medical device. For example, in some embodiments the interventional element 100 may be a stent and/or stent retriever, such as Medtronic's Solitaire' Revascularization Device, Stryker Neurovascular's Trevo^{®} ProVue^{™} Stentriever, or other suitable devices. In some embodiments, the interventional element 100 may be a coiled wire, a weave, and/or a braid formed of a plurality of braided filaments. Examples of suitable interventional elements 100 include any of those disclosed in U.S. Patent No. 7,300,458, filed November 5, 2007, U.S. Patent No. 8,940,003, filed November 22, 2010, U.S. Patent No. 9,039,749, filed October 1, 2010, and U.S. Patent No. 8,066,757, filed December 28, 2010.

In some embodiments, the interventional element 100 is a mesh structure (e.g., a braid, a stent, etc.) formed of a superelastic material (e.g., Nitinol) or other resilient or self-expanding material configured to self-expand when released from the third catheter 12. The mesh structure may include a plurality of struts 101 and open spaces 103 between the struts 101. In some embodiments, the struts 101 and spaces 103 may be situated along the longitudinal direction of the interventional element 100, the radial direction, or both.

As depicted in Figure 2A, the interventional element 100 may comprise a working length WL portion and a non-working length NWL portion. The portion of the interventional element 100 in the working length WL may be configured to interlock, capture, and/or engage a thrombus. The portion of the interventional element 100 in the non-working length NWL may contact thrombotic material in use, but is configured to perform a function that renders it ineffective or less effective than the working length WL portion for interlocking, capturing, and/or engaging with a thrombus. In some embodiments, such as that shown in Figure 2A, a distal terminus of the working length WL portion is proximal of the distal terminus of the interventional element 100 (i.e., the working length WL portion is spaced apart from the distal terminus of the interventional element 100), and the non-working length NWL portion is disposed between the working length WL and the band 220 and/or the distal end of the core member 11.

With continued reference to Figure 2A, in some embodiments, the non-working length NWL portion of the interventional element 100 can be coated with a non-conductive or insulative material (e.g., Parylene, PTFE, or other suitable non-conductive coating) such that the coated region is not in electrical contact with the surrounding media (e.g., blood). As a result, the current carried by the core member 11 to or from the interventional element 100 is only exposed to the surrounding media along the working length WL portion of the interventional element 100. This can advantageously concentrate the electrically enhanced attachment effect along the working length WL of the interventional element 100, where it is most useful, and thereby combine both the mechanical interlocking provided by the working length WL and the electrical enhancement provided by the delivered electrical signal. In some embodiments, a distal region of the interventional element 100 (e.g. distal of the working length WL) may likewise be coated with a non-conductive material (e.g., Parylene, PTFE, or other suitable non-conductive coating), leaving only a central portion or the working length WL of the interventional element 100 having an exposed conductive surface.

In some embodiments, the interventional element 100 may include a conductive material positioned on some or all of its outer surface. The conductive material, for example, can be gold and/or another suitable conductor that has a conductivity greater than (or a resistivity less than) that of the material comprising the interventional element 100. The conductive material may be applied to the interventional element 100 via electrochemical deposition, sputtering, vapor deposition, dip-coating, and/or other suitable means.

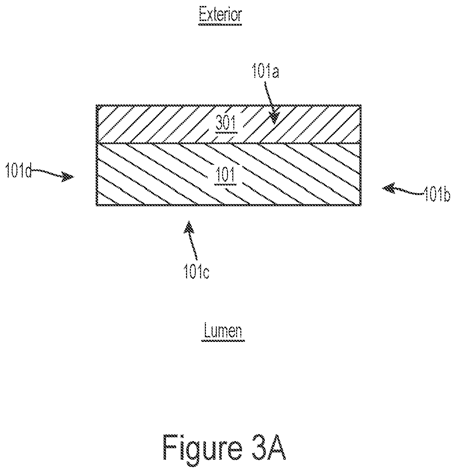

Figure 3A is a cross-sectional view of a strut 101 of the interventional element 100 having a material 301 disposed thereon. In various embodiments, the material 301 can be electrically insulative or conductive, as the case may be, to achieve the desired electrical properties. In some embodiments, different materials 301 can be applied in different portions of the interventional element 100. Moreover, in some embodiments, a given material 301 can be deposited with varying thickness across the interventional element 100.

Although the strut 101 shown in Figure 3A has a generally square or rectangular cross-sectional shape, in some embodiments the interventional element 100 includes one or more struts or filaments having other cross-sectional shapes (e.g., circle, oval, etc.). The strut 101 has a surface comprised of an outer portion 101a facing away from a lumen or a central longitudinal axis of the interventional element 100, an inner portion 101c facing toward the lumen or central longitudinal axis, and side portions 101b and 101d extending between the outer and inner portions 101a, 101c. In some embodiments, such as that shown in Figure 3A, the material 301 may be disposed only at the outer portion 101a of the strut 101 and the inner and side portions 101b-d may be exposed, or otherwise not in contact with or covered by the material 301. In some embodiments, the material 301 may be disposed only on the inner portion 101c of the surface of the strut 101, only on one of the side portions 101b, 101d, or on any combination of the surface portions 101a-d.

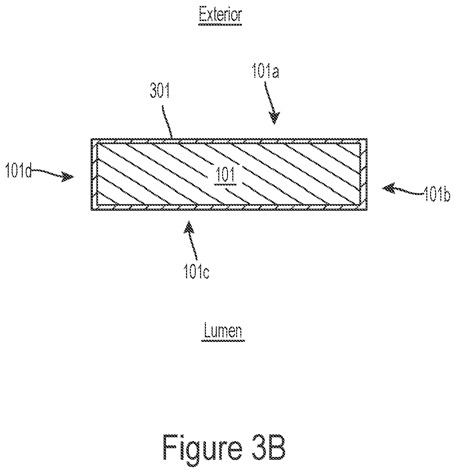

Figure 3B illustrates another example cross-sectional view in which a strut 101 is surrounded on all surface portions 101a-d with a material 301. In such cases, the material 301 can be an electrically insulative or conductive coating that is selected, as the case may be, to achieve the desired electrical properties. In some embodiments, the material 301 is the result of surface treatment of the interventional element including the strut 101. For example, the interventional element can be anodized to create an oxide layer and/or to increase a thickness of a pre-existing oxide layer over the interventional element (e.g., substantially surrounding strut 101). The thickness of the material 301 (e.g., an oxide layer) can impact the local conductivity of the strut 101, for example with a thicker oxide layer providing a lower conductivity than a thinner oxide layer. As such, by controlling the oxide layer and varying its thickness over different portions of the interventional element, a desired overall distribution of electrical conductivity over the surface of the interventional element can be achieved. In at least some embodiments, the material 301 can have a relatively greater thickness in a proximal portion of the interventional element (e.g., along some or all of the non-working length NWL and/or a proximal portion of the working length WL) and a relatively smaller thickness a distal portion of the interventional element (e.g., along some or all of the working length WL).

In some aspects of the present technology, the material 301 is disposed only on the working length WL portion of the interventional element 100 such that the proximal and distal end portions 100a, 100b of the interventional element 100 are exposed. In embodiments in which the material 301 is electrically conductive, the material can have a lower or even much lower resistance than the underlying material comprising the interventional element 100, and therefore current delivered to the interventional element 100 may be concentrated along the working length WL portion. In several of such embodiments, the conductive material 301 may be disposed only on the outer portion 101a of the strut surface along the working length WL portion. In other embodiments, the conductive material 301 may be disposed on all or a portion of the strut surface along all or a portion of the length of the interventional element 100.

In some embodiments, a first portion of the interventional element 100 is covered by a conductive material and a second portion of the interventional element 100 is covered by an insulative or dielectric material (e.g., Parylene and/or any other electrically insulative material or polymer). For example, in some embodiments the outer portion 101a of the strut surface is covered by a conductive material while an inner portion 101c of the strut surface is covered by an insulative material. In some embodiments, the working length WL portion of the interventional element 100 may be covered by a conductive material while the non-working length NWL portion is covered by an insulative material. In some embodiments, the conductive material may be disposed on all or a portion of the strut surface along all or a portion of the length of the interventional element 100, and the insulative material may be disposed on those portions of the strut surface and/or working length not covered by the conductive material.

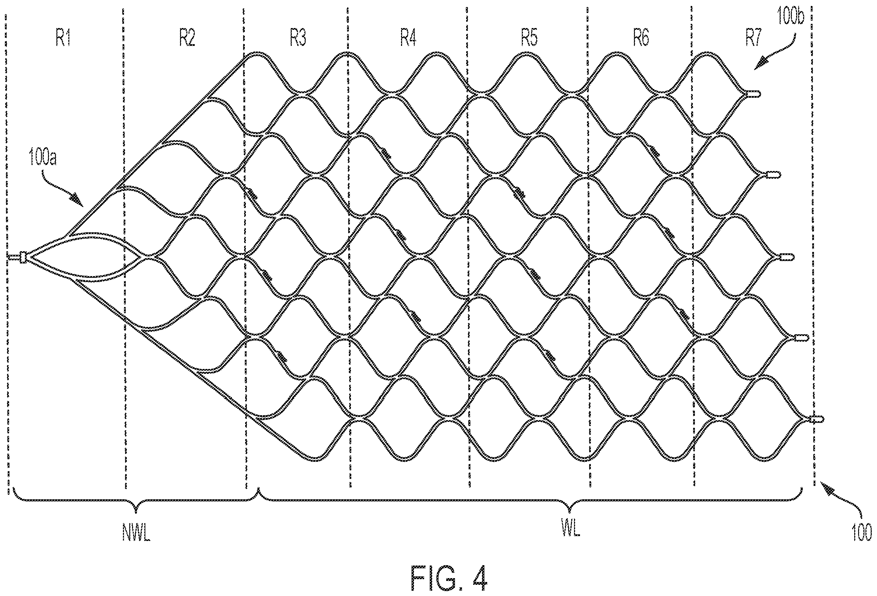

Figure 4 illustrates a plan view of an interventional element 100 having a proximal end portion 100a and a distal end portion 100b. As described previously with respect to Figure 2A, the interventional element 100 can include a working length WL and a non-working length NWL. As shown in Figure 4, the interventional element 100 includes a plurality of regions R1-R7 which are arranged adjacent one another in a proximal-distal direction. As described elsewhere herein, it may be desirable to vary the surface electrical properties of the interventional element among different regions of the interventional element 100. For example, a surface treatment (e.g., anodization), coating, strut geometry, or other feature can be selected and/or varied to achieve varying electrical properties among the different regions. In the illustrated example, a surface electrical conductivity can vary among the different regions R1-R7, for example with a surface conductivity increasing in the distal direction, e.g., such that the surface conductivity in region R7 is greater than that of region R6, which is greater than that of region R5, which is greater than that of region R4, and so on down to region R1 which has the lowest surface conductivity among the regions R1-R7. Accordingly, for an interventional element 100 having N regions arranged in the proximal-to-distal direction similar to Figure 4, the surface conductivity SC of any given region *Rₓ* can be related to the surface conductivity SC of a proximally-adj acent region *Rₓ₋₁* by the relation [*SC_{Rx}* > *SC*_{*Rx*-1}] where x is a positive integer ranging from 2 to *N,* and *SC_{R1}* is no less than zero. In some embodiments, such a gradation can be achieved by varying a thickness of an insulative material disposed over the surface of the interventional element among the different regions R1-R7. For example, the insulative material can have a first thickness in the proximalmost region R1. The insulative material can have a second thickness less than the first in R2, and a third thickness less than the second in R3, etc. In this manner, each subsequent distal region can have a thickness of the insulative material (e.g., an oxide layer, which can optionally be provided by anodization) that is thinner or smaller than the more proximal regions. As a result, the surface electrical conductivity of each region can increase in the distal direction. This arrangement can counteract the tendency for electrical current to concentrate along a proximal portion of the interventional element (e.g., along the non-working length NWL).

Although discrete regions R1-R7 are illustrated here, in other embodiments the thickness of the insulative material can be varied in a continuous fashion without well-defined steps or transitions between adjacent regions. Additionally, the regions R1-R7 shown here are illustrative only, and in various embodiments the interventional element can be subdivided into different numbers or arrangements of regions (e.g., fewer or greater regions, regions arranged along a circumferential direction perpendicular to the proximal-distal variations shown in Figure 4, regions of varying size and shape, etc.) In some embodiments, the thickness of the insulative material may not monotonically increase or decrease along a proximal-distal direction, but rather may be arranged in other fashions. For example, a thickness of the insulative material may be greatest in region R4, with regions R3 and R5 each having lower thicknesses, and regions R2 and R6 lower thicknesses still, and regions R1 and R7 with little or no insulative material thereon. Various other configurations are possible and can be selected to achieve the desired electrical properties of the interventional element.

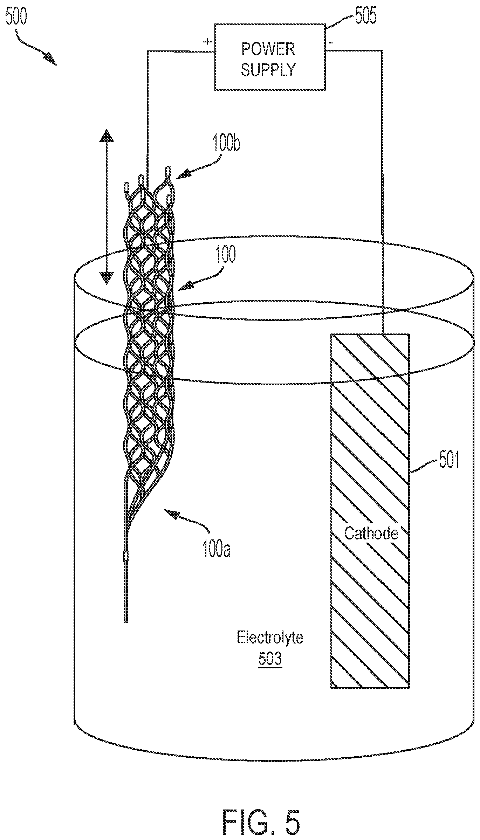

Figure 5 is a schematic illustration of anodizing an interventional element 100 in accordance with the present technology. Anodization is a process for surface treating a metallic material to create and/or increase a thickness of an overlying oxide layer. As shown, an anodization system 500 includes an interventional element 100 and a cathode 501 each at least partially submerged in an electrolyte solution 503. The interventional element 100 is electrically coupled to the positive terminal of a power supply 505 and the cathode 501 is electrically coupled to the negative terminal of the power supply 505, such that a voltage is applied between the interventional element 100 and the cathode 501. Upon application of voltage via the power supply 505, oxygen ions are released from the electrolyte to combine with atoms of interventional element material at the surface thereof, thereby forming a metal-oxide layer over the surface of the interventional element (at least any portion of the interventional element that is immersed or submerged within the electrolyte 503). In the case of an interventional element 100 made of Nitinol, a resulting oxide layer can include a Ti-Ni-O oxide (e.g., Ti₄Ni₂O). In many instances, oxidation may naturally occur in the presence of air such that a pre-anodized interventional element 100 already includes a thin film of a metal-oxide layer. In such instances, an anodization process can result in a controlled increase in the thickness of the naturally occurring oxide layer over the interventional element 100.

The rate of growth of the oxide layer can be driven by a number of factors, including the size, material composition, and relative placement of the cathode 501 within the electrolyte solution 503, as well as the material composition and volume of the electrolyte solution 503. Additionally, the rate of growth of the oxide layer can be driven by the voltage applied via the power source 505 and the amount of time that anodization is performed (e.g., the amount of time that voltage is applied and/or the amount of time that the interventional element 100 is immersed within the electrolyte 503). By moving the interventional element 100 into and out of the electrolyte 503, it is possible to anodize one portion (e.g., proximal end portion 100a) to a greater extent than another portion (e.g., distal end portion 100b). By gradually removing the interventional element 100 from the electrolyte 503, the portions removed earlier will generally have thinner oxide layers than the portions removed later. This can be used to achieve discrete "steps" with different oxide thicknesses by moving the interventional element 100 by discrete amounts and then leaving it in position for a period of time before moving it further. Alternatively, a more continuous gradient or transition can be achieved by slowly but continuously moving the interventional element 100 out of the electrolyte 503, thereby producing a gradually increasing thickness of the oxide layer along the axis by which the interventional element 100 was removed from the electrolyte 503. In the illustrated example, the interventional element 100 can be removed along the distal-proximal direction, such that the proximal end portion 100a can have a thicker oxide layer than the distal end portion 100b. In some embodiments, at least a portion of the interventional element 100 is not immersed within the electrolyte 503 (e.g., the distal end portion 100b), and therefore is not subject to anodization, while other portions of the interventional element 100 are anodized.

In various embodiments, the thickness of the oxide layer can range from 0 (i.e., in some regions there may be no oxide layer formed at all) to about 200 nm (from 0 to about 2000 Angstroms) or more. The thickness of the oxide layer can be driven at least in part by the voltage applied via the power supply 505, with increasing voltage resulting in increasing thickness of the oxide layer. Additionally, in some embodiments the oxide layer can have an apparent color that varies with thickness, for example appearing more silver or brown at lower thicknesses and yellow, pink, blue, and green at greater thicknesses. In some embodiments this can permit visual inspection for non-destructive evaluation of an oxide layer thickness.

Any suitable materials for the cathode 501 and electrolyte solution 503 can be selected to achieve the desired performance. Example materials for the cathode 501 include conductive metals such as platinum, aluminum, stainless steel, titanium, and alloys thereof, or any other suitable material. In some embodiments, the electrolyte 503 can be selected to be substantially non-corrosive to the interventional element 100, which as noted above may be made of Nitinol or other suitable metallic material. Any suitable electrolyte 503 can be used. Examples include H₂SO₄, Na₂SO₄, CH₃COOH, H₃PO₄, and HF.

The resulting oxide layer can take the form of an amorphous surface layer that still allows for electron exchange, albeit at a lower level than the bare underlying metal of the interventional element 100 due to inherent insulative characteristics of the amorphous surface layer. This resistance and alteration to the electron exchange will vary depending on the oxide layer thickness. In some embodiments, to achieve a current density distribution that is more uniform across the surface of the interventional element, or even to allow for increased current density at a more distal portion of the interventional element, the anodization process can be performed in a manner such that the interventional element 100 is gradually removed from the anodizing electrolyte 503 to form the desired charge gradient and/or conductivity gradient (e.g., with a greater thickness in a proximal portion of the interventional element 100 than in a distal portion of the interventional element 100).

The addition of the oxide layer can also reduce the production of hydrogen and chlorine by-product bubbles when current is applied to the interventional element while in the presence of aqueous chloride media such as blood. As noted previously, hydrogen and chlorine gas bubbles can form when surface charge is concentrated over a small area of the interventional element while in the presence of blood. By using an oxide layer of varying thickness to achieve a more uniform or otherwise favorable surface charge distribution, the production of hydrogen and chlorine gas bubbles can be reduced or eliminated. In addition to achieving the desired electrical properties, anodization can increase the corrosion resistance of the interventional element 100.

Figures 6-8 illustrate additional embodiments of interventional elements 600, 700, 800 that can be configured to carry electrical current for use in thrombectomy procedures or other suitable applications. In various embodiments, the interventional elements 600, 700, and 800 can be coupled to core members and provided with electrode arrangements, current generator configurations, etc. that can be similar to the various embodiments of the interventional element 100 and associated electrode arrangements described herein, except as otherwise specified. For example, any of the interventional elements 600, 700, 800 shown herein can include a surface treatment (e.g., anodization) or coating to modify the electrical properties of its surface to achieve the desired overall electrical properties. In various embodiments, a proximal region or other non-working length of the interventional elements 600, 700, 800 can be coated or surface-treated to decrease a surface conductivity in those region(s). This arrangement can favorably distribute more surface charge to distal regions and/or working lengths of the interventional elements 600, 700, 800.

As shown in Figures 6-8, the interventional elements can take a number of different forms while benefiting from the electrically enhanced adhesion to clot material provided by the appropriate surface properties (e.g., by anodizing and/or coating some or all of the interventional element). For example, with respect to Figure 6, the interventional element 600 is a clot retrieval device with an inner tubular member 601 and a multi-segment outer expandable member 603 having a greater diameter than the inner tubular member. The outer member 603 can have radially outwardly extending struts 605 defining inlet mouths 607 configured to receive clot material therein. With respect to Figure 7, the interventional element 700 is another example of a clot retrieval device, in this instance comprising a plurality of interlinked cages 701a-e having an atraumatic leading surface 703. Each of the cages 701a-e can be configured to expand radially outwardly to engage a thrombus. Figure 8 illustrates another example interventional element 800 in the form of a clot retrieval device, here comprising a coiled or helical member 801 configured to be expanded into or distal to a thrombus, thereby engaging the thrombus between turns of the coil 801 and facilitating removal from the body. In addition to these illustrative examples, the interventional element can take other forms, for example a removal device, a thrombectomy device, a retrieval device, a braid, a mesh, a laser-cut stent, or any suitable structure.

### IV. Select Methods of Use

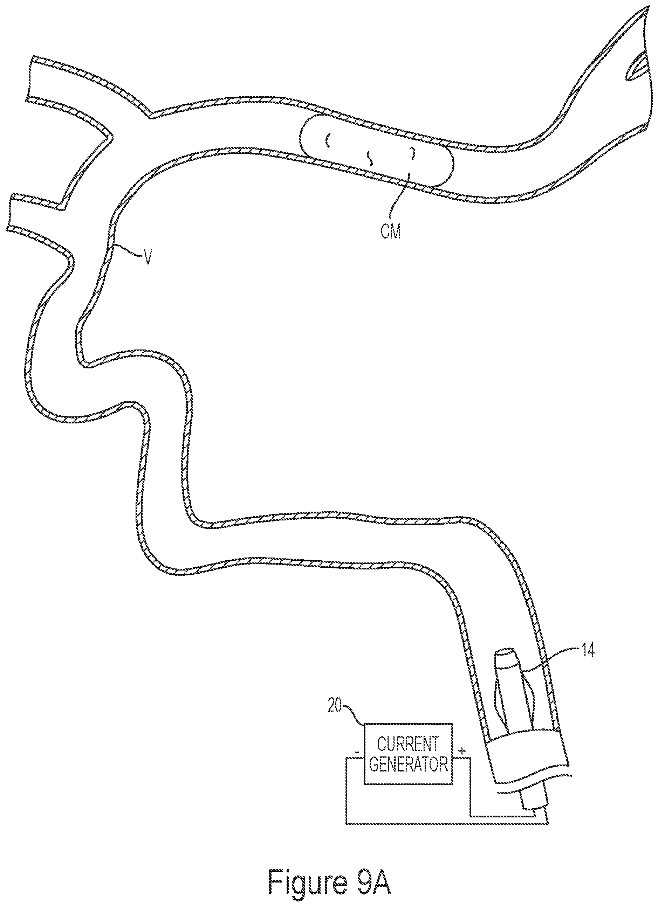

Figures 9A-9G illustrate a method of removing clot material CM from the lumen of a blood vessel V using the treatment system 10 of the present technology. As shown in Figure 9A, the first catheter 14 can be advanced through the vasculature and positioned within the blood vessel such that a distal portion of the first catheter 14 is proximal of the clot material CM. As shown in Figure 4B, the second catheter 13 may be advanced through the first catheter 14 until a distal portion of the second catheter 13 is at or proximal to the clot material CM. Next, the third catheter 12 may be advanced through the second catheter 13 so that a distal portion of the third catheter 12 is positioned at or near the clot material CM. In some embodiments, the third catheter 12 may be positioned such that a distal terminus of the third catheter 12 is distal of the clot material CM. The interventional element 100 may then be advanced through the third catheter 12 in a low-profile configuration until a distal terminus of the interventional element 100 is at or adjacent the distal terminus of the third catheter 12.

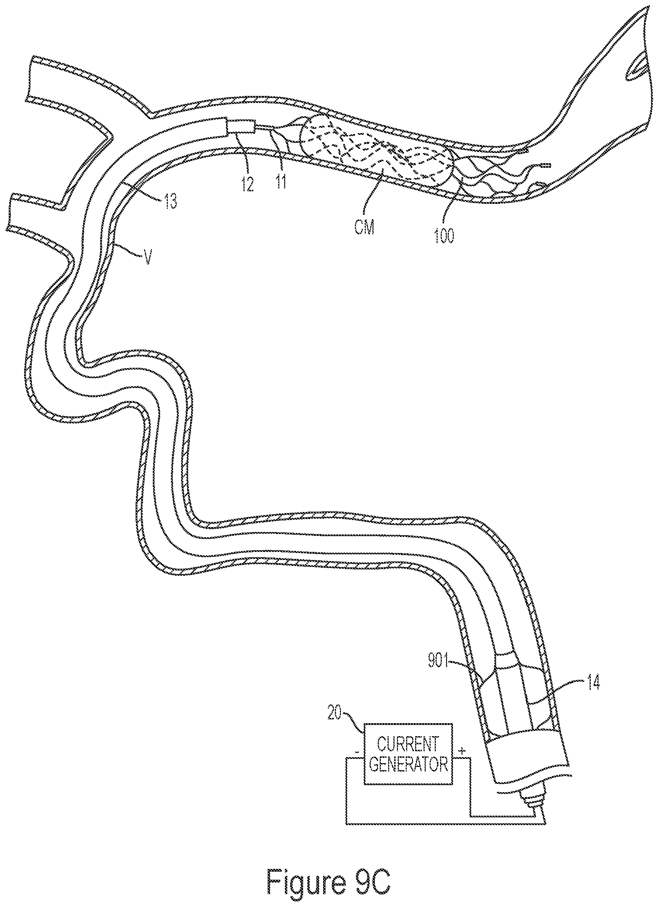

As shown in Figure 9C, the third catheter 12 may be withdrawn proximally relative to the interventional element 100 to release the interventional element 100, thereby allowing the interventional element 100 to self-expand within the clot material CM. As the interventional element 100 expands, the interventional element 100 engages and/or secures the surrounding clot material CM, and in some embodiments may restore or improve blood flow through the clot material CM by pushing open a blood flow path therethrough. In some embodiments, the interventional element 100 may be expanded distal of the clot material CM such that no portion of the interventional element 100 is engaging the clot material CM while the interventional element 100 is in the process of expanding toward the vessel wall. In some embodiments, the interventional element 100 is configured to expand into contact with the wall of the vessel V, or the interventional element 100 may expand to a diameter that is less than that of the blood vessel lumen such that the interventional element 100 does not engage the entire circumference of the blood vessel wall.

Once the interventional element 100 has been expanded into engagement with the clot material CM, the interventional element 100 may grip the clot material CM by virtue of its ability to mechanically interlock with the clot material CM. The current generator 20, which is electrically coupled to the proximal end of the core member 11, can deliver a current to the interventional element 100 before or after the interventional element 100 has been released from the third catheter 12 into the blood vessel and/or expanded into the clot material CM. The interventional element 100 can be left in place or manipulated within the vessel V for a desired time period while the electrical signal is being delivered. Positive current delivered to the interventional element 100 can attract negatively charged constituents of the clot material CM, thereby enhancing the grip of the interventional element 100 on the clot material CM. This allows the interventional element 100 to be used to retrieve the clot material CM with reduced risk of losing grip on the thrombus or a piece thereof, which can migrate downstream and cause additional vessel blockages in areas of the brain that are more difficult to reach.

In some methods of the present technology, a guidewire (not shown) may be advanced to the treatment site and pushed through the clot material CM until a distal portion of the guidewire is distal of the clot material CM. The guidewire may be advanced through one or more of the catheters 12-14 and/or one or more of the catheters 12-14 may be advanced over the guidewire. The guidewire may be insulated along at least a portion of its length (e.g., with Parylene, PTFE, etc.), with exposed portions permitting electrical communication with the current generator 20 and the interventional element 100. For example, in some embodiments a distal portion of the guidewire may be exposed, and the guidewire may be positioned at the treatment site such that the exposed portion of the guidewire is distal of the clot material CM. A proximal end of the guidewire may be coupled to the current generator such that the exposed portion of the guidewire functions as a return electrode. In some embodiments, the guidewire may be coupled to the positive terminal of the power source and the exposed portion functions as a delivery electrode. The guidewire may be used as a delivery or return electrode with any delivery or return electrode carried by any component of the treatment system (e.g., one or more of the first-third catheters 14, 13, 12, the interventional element 100, etc.).

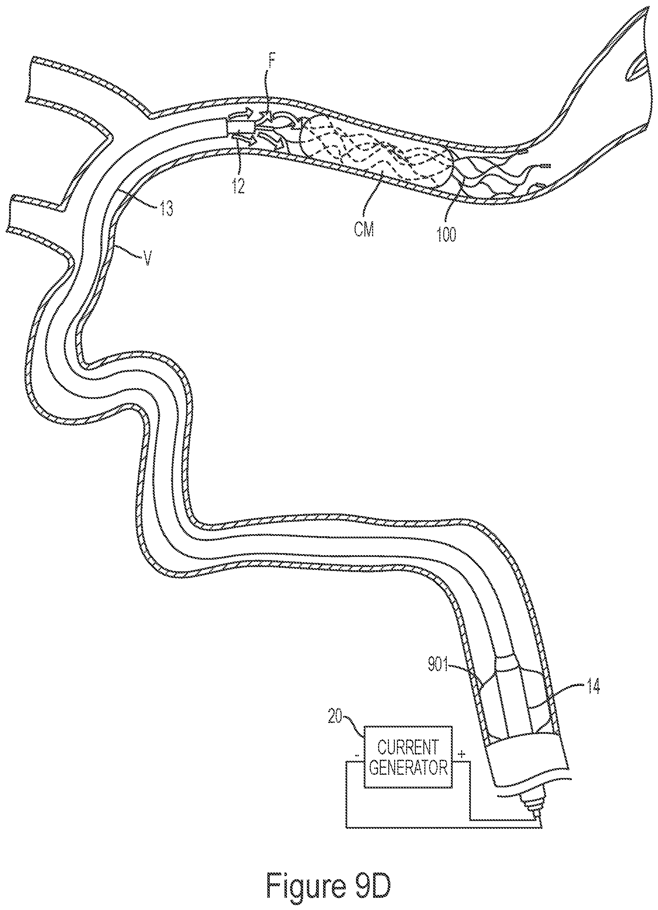

Figures 9D-9F illustrate optional processes that may be performed before, during, and/or after deployment of the interventional element 100. With reference to Figure 9D, in some methods fluid F may be delivered to the treatment site via the second catheter 13 and/or third catheter 12 while current is being delivered to the interventional element 100. Fluid delivery may occur before or while the interventional element 100 is engaging the thrombus, and may coincide with the entire duration of current delivery or just a portion thereof.

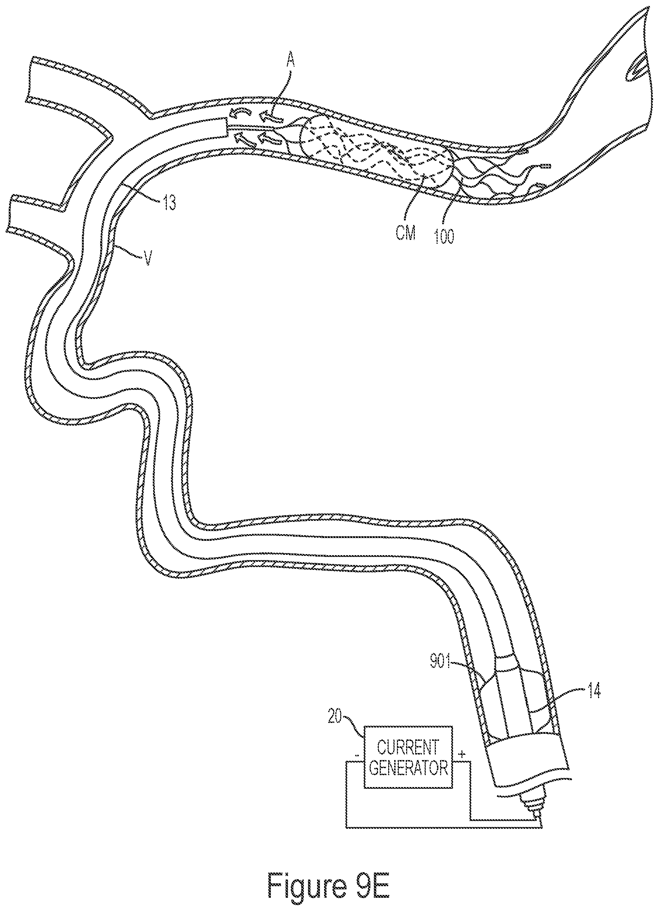

Referring now to Figure 9E, in some instances, aspiration may be applied to the treatment site via the second catheter 13. For example, following deployment of the interventional element 100, the third catheter 12 can be retracted and removed from the lumen of the second catheter 13. The treatment site can then be aspirated via the second catheter 13, for example via a suction source such as a pump or syringe coupled to a proximal portion of the second catheter 13. In some embodiments, following expansion of the interventional element 100, the treatment site is aspirated concurrently with supplying electrical energy to the interventional element 100 via the current generator 20. By combining aspiration with the application of electrical energy, any newly formed clots (e.g., any clots formed that are attributable at least in part to the application of electrical energy), or any clot pieces that are broken loose during the procedure, can be pulled into the second catheter 13, thereby preventing any such clots from being released downstream of the treatment site. As a result, concurrent aspiration may permit the use of higher power or current levels delivered to the interventional element 100 without risking deleterious effects of new clot formation. Additionally, aspiration can capture any gas bubbles formed along the interventional element 100 or marker band 220 (Figure 2A) during application of electrical energy to the interventional element 100, which can improve patient safety during the procedure.

In some embodiments, aspiration is applied while the interventional element 100 is retracted into the second catheter 13. Aspiration at this stage can help secure the clot material CM within the second catheter 13 and prevent any dislodged portion of the clot material CM from escaping the second catheter 13 and being released back into the vessel V. In various embodiments, the treatment site can be aspirated continuously before, during, or after delivering electrical signals to the interventional element 100 as well as before, during, or after retraction of the interventional element 100 into the second catheter 13.

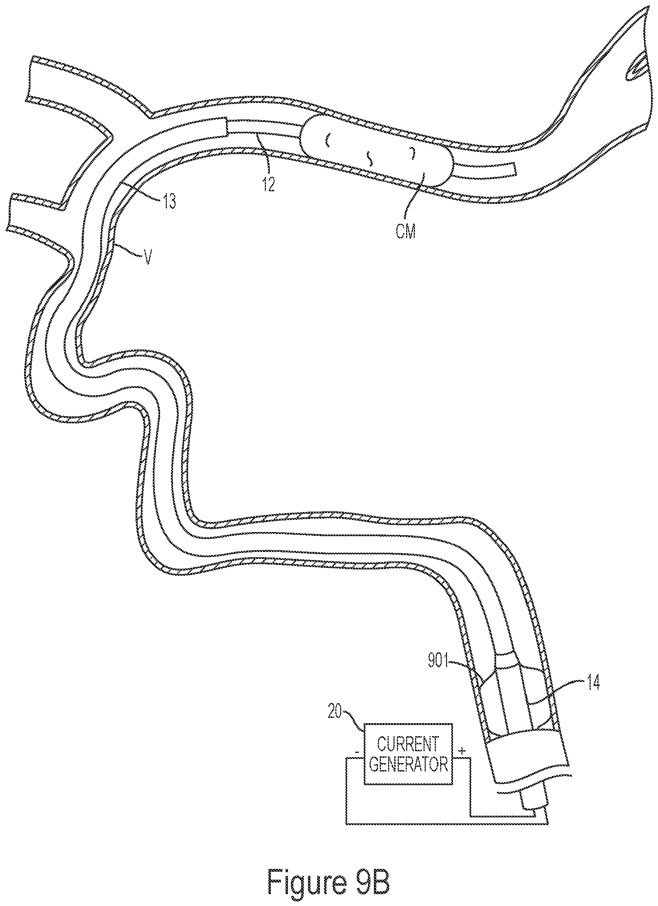

With reference to Figures 9B-9F, at any time before, during, and/or after deployment of the interventional element 100, a flow arrest element may be deployed within the blood vessel proximal of the clot material CM to partially or completely arrest blood flow to the treatment site. For example, as shown in Figures 6B-6F, the first catheter 14 may be a balloon guide catheter having a balloon 901 at its distal portion. The balloon 901 may be configured to inflate or expand into apposition with the surrounding blood vessel wall, thereby at least partially arresting blood flow distal to the balloon 901. In some embodiments, the flow arrest element can have other forms or configurations suitable for partially or completely arresting blood flow within the vessel V.

In some methods, the flow arrest element may be deployed at a location along the blood vessel proximal of the clot material CM (for example, at a proximal portion of the internal carotid artery) and may remain inflated as the interventional element 100 is deployed and eventually withdrawn to remove the thrombus. For example, Figures 9B-9F show the balloon 901 blocking flow from a portion of the artery proximal of the balloon toward the interventional element 100 and treatment area, while the second catheter 13 and third catheter 12 are positioned at the treatment site (Figure 9B), while the interventional element 100 is expanded within the clot material CM (Figure 9C), while fluid is infused at the treatment site (Figure 9D), and while aspiration is applied at the treatment site (Figure 9E). Although the balloon 901 is shown in an expanded state in each of Figures 9B-9F, it will be appreciated that the balloon 901 may be in an unexpanded state and/or deflated at any time throughout the procedure to allow blood flow.

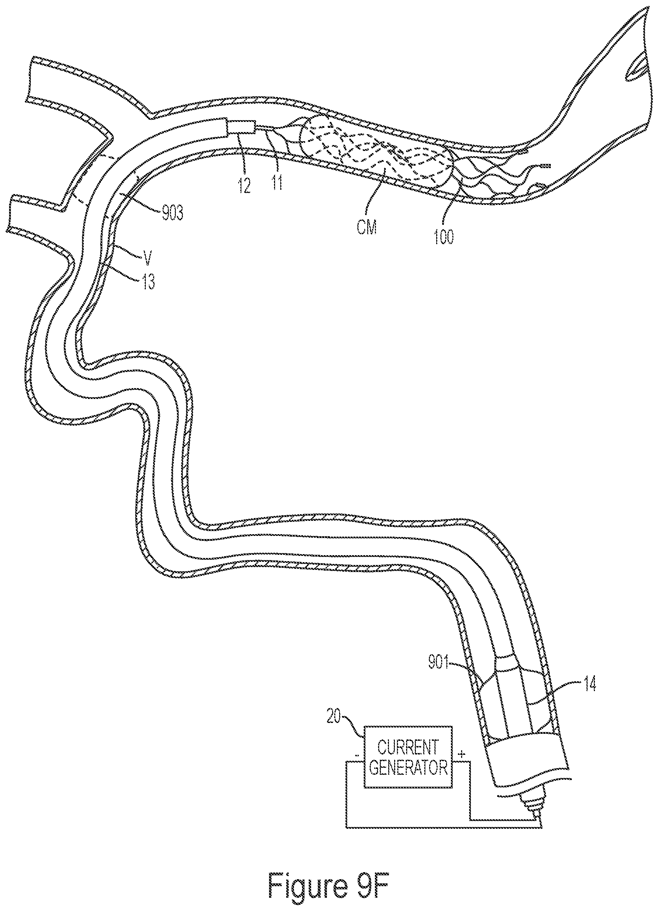

As shown in Figure 9F, in some embodiments the flow arrest element may be a balloon 903 coupled to the second catheter 13 (such as a distal access catheter). In such embodiments, the first catheter 14 may not include a flow arrest element such that flow arrest is achieved via deployment of the flow arrest element coupled to the second catheter 13. For example, in such embodiments, the first catheter 14 may be a sheath or support catheter. The balloon 903 may be inflated at a location distal of the distal end of the first catheter 14, closer to the thrombus. In some methods, the flow arrest element may be deflated and inflated several times throughout the procedure.

At least while the interventional element 100 is deployed and engaging the thrombus CM, electric current may be delivered to the interventional element 100 to positively charge the interventional element 100, thereby enhancing clot adhesion to the interventional element 100. As previously discussed, the inventors have observed improved electrically enhanced clot adhesion in the absence of blood flow. As such, it may be especially beneficial to arrest blood flow (e.g., via a flow arrest element on the first or second catheter 14, 13) while the interventional element 100 is charged, and while expanding the interventional element 100 within the thrombus and/or when withdrawing the thrombus proximally.

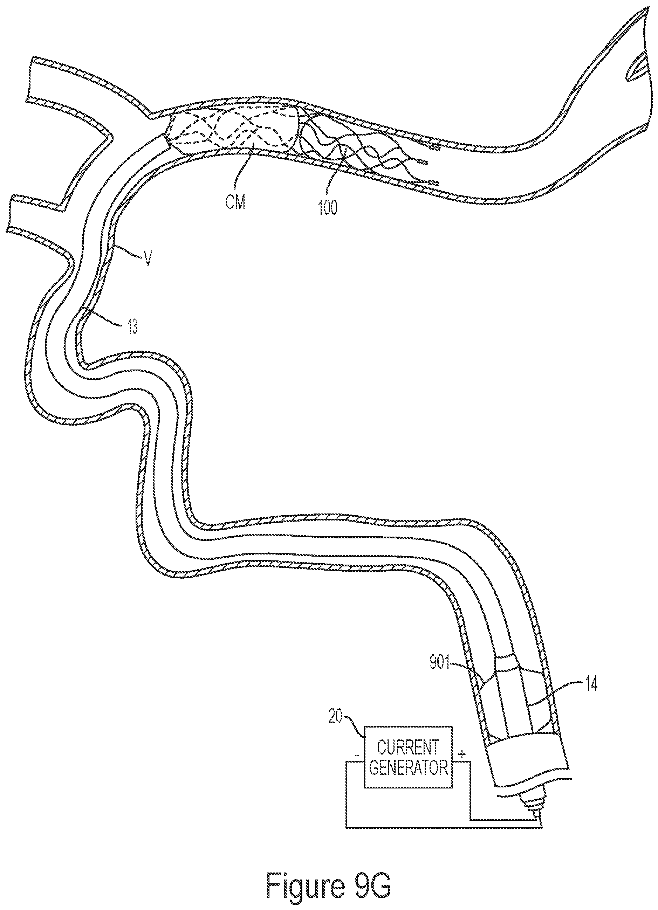

With reference to Figure 9G, while the interventional element 100 is engaged with the clot material CM, the clot material CM can be removed. For example, the interventional element 100, with the clot material CM gripped thereby, can be retracted proximally (for example, along with the second catheter 13 and, optionally, the third catheter 12). The second catheter 13, interventional element 100, and associated clot material CM may then be withdrawn from the patient, optionally through one or more larger surrounding catheters. During this retraction, the interventional element 100 can grip the clot material CM electrically and/or electrostatically, e.g., via the application of current from a current generator as discussed herein. (As used herein with reference to gripping or retrieving thrombus or other vascular/luminal material, or to apparatus for this purpose, "electrical" and its derivatives will be understood to include "electrostatic" and its derivatives.) Accordingly, the interventional element 100 can maintain an enhanced or electrically and/or electrostatically enhanced grip on the clot material CM during retraction. In other embodiments, the current generator 20 may cease delivery of electrical current or signals to the interventional element 100 prior to retraction of the interventional element 100 with respect to the vessel V. In some embodiments, the interventional element 100 and clot material CM form a removable, integrated thrombus-device mass wherein the connection of the thrombus to the device is electrically enhanced, e.g. via the application of current as discussed herein.

### IV. Select Embodiments of Waveforms for Electrically Enhanced Retrieval

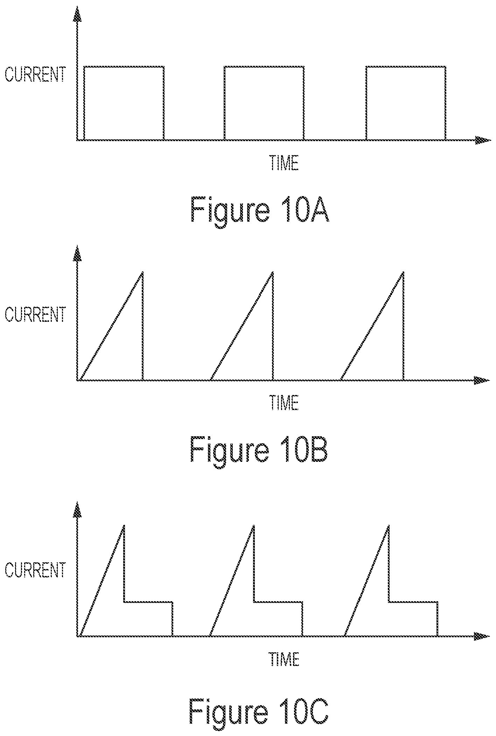

Figures 10A-10E show various electrical waveforms for use with the treatment systems of the present technology. Although the waveforms and other power delivery parameters disclosed herein can be used with the devices and methods described above with respect to Figures 1A-9G, the waveforms and other parameters are also applicable to other device configurations and techniques. For example, the return electrode can be provided along the catheter wall, as a separate conductive member extending within the catheter lumen, as a needle electrode provided elsewhere in the body, etc. In each of these device configurations, the power delivery parameters and waveforms can be beneficially employed to promote clot adhesion without damaging surrounding tissue. Additionally, although the waveforms and other power delivery parameters disclosed herein may be used for treating a cerebral or intracranial embolism, other applications and other embodiments in addition to those described herein are within the scope of the technology. For example, the waveforms and power delivery parameters disclosed herein may be used to electrically enhance removal of emboli from body lumens other than blood vessels (e.g., the digestive tract, etc.) and/or may be used to electrically enhance removal of emboli from blood vessels outside of the brain (e.g., pulmonary blood vessels, blood vessels within the legs, etc.).

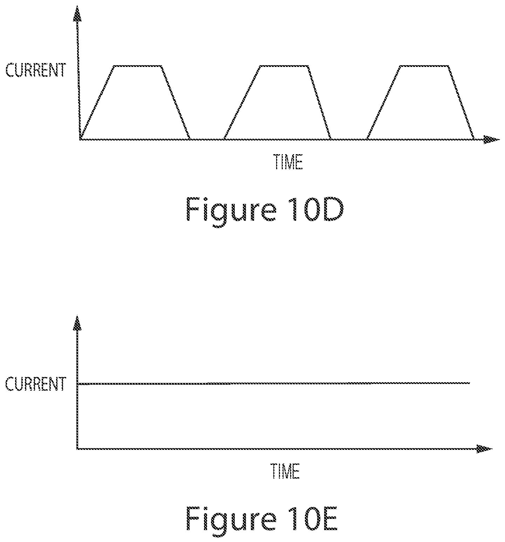

While applying a continuous uniform direct current (DC) electrical signal (as shown in Figure 10E) to positively charge the interventional element and/or aspiration catheter can improve attachment to the thrombus, this can risk damage to surrounding tissue (e.g., ablation), and sustained current at a relatively high level may also be thrombogenic (i.e., may generate new clots). For achieving effective clot-grabbing without ablating tissue or generating substantial new clots at the treatment site, periodic waveforms have been found to be particularly useful. Without wishing to be bound by theory, the clot-adhesion effect appears to be most closely related to the peak current of the delivered electrical signal. Periodic waveforms can advantageously provide the desired peak current without delivering excessive total energy or total electrical charge. Periodic, non-square waveforms in particular are well suited to deliver a desired peak current while reducing the amount of overall delivered energy or charge as compared to either uniform applied current or square waveforms.

Figures 10A-10D illustrate various periodic waveforms that can be used with the devices and methods described above with respect to Figures 1A-9G, as well as with other devices and techniques. Figure 10E illustrates a continuous uniform DC electrical signal which can also be used in some embodiments. Referring to Figures 10A-10D, electrical power can be delivered according to these waveforms as pulsed direct current. Figures 10A and 10B illustrate periodic square and triangular waveforms, respectively. These two waveforms have the same amplitude, but the triangular waveform is able to deliver the same peak current as the square waveform, with only half of the total charge delivered, and less total energy delivered. Figure 10C illustrates another pulsed-DC or periodic waveform which is a composite of a square waveform and a triangular waveform. This superposition of a triangular waveform and a square waveform shown in Figure 10C delivers additional efficacy compared to the triangular waveform of Figure 10B while nonetheless delivering less overall energy than the square waveform of Figure 10A. This is because the delivered energy is proportional to the square of current and the brief high peak in the composite waveform of Figure 10C ensures that current is supplied without dispensing excessive energy. Figure 10D illustrates yet another non-square waveform, in this case a trapezoidal waveform in which "ramp-up" and "ramp-down" portions at the beginning and end of each pulse provide periods of reduced current compared to square waveforms. In other embodiments, different non-square waveforms can be used, including a superposition of a square waveform with any non-square waveform, depending on the desired power delivery characteristics.

The waveform shape (e.g., pulse width, duty cycle, amplitude) and length of time can each be selected to achieve desired power delivery parameters, such as overall electrical charge, total energy, and peak current delivered to the interventional element and/or catheter. In some embodiments, the overall electrical charge delivered to the interventional element and/or catheter can be between about 30-1200 mC, or between about 120-600 mC. According to some embodiments, the total electrical charge delivered to the interventional element and/or catheter may be less than 600 mC, less than 500 mC, less than 400 mC, less than 300 mC, less than 200 mC, or less than 100 mC.

In some embodiments, the total energy delivered to the interventional element and/or aspiration catheter can be between about 0.75-24,000 mJ, or between about 120-24,000 mJ, or between about 120-5000 mJ. According to some embodiments, the total energy delivered to the interventional element and/or aspiration catheter may be less than 24,000 mJ, less than 20,000 mJ, less than 15,000 mJ, less than 10,000 mJ, less than 5,000 mJ, less than 4,000 mJ, less than 3,000 mJ, less than 2000 mJ, less than 1,000 mJ, less than 900 mJ, less than 800 mJ, less than 700 mJ, less than 600 mJ, less than 500 mJ, less than 400 mJ, less than 300 mJ, or less than 200 mJ, or less than 120 mJ, or less than 60 mJ, or less than 48 mJ, or less than 30 mJ, or less than 12 mJ, or less than 6 mJ, or less than 1.5 mJ.

In some embodiments, the peak current delivered can be between about 0.5-20 mA, or between about 0.5-5 mA. According to some embodiments, the peak current delivered may be greater than 0.5 mA, greater than 1 mA, greater than 1.5 mA, greater than 2 mA, greater than 2.5 mA, or greater than 3 mA.

The duration of power delivery is another important parameter that can be controlled to achieve the desired clot-adhesion effects without damaging tissue at the treatment site or generating new clots. In at least some embodiments, the total energy delivery time can be no more than 1 minute, no more than 2 minutes, no more than 3 minutes, no more than 4 minutes, or no more than 5 minutes. According to some embodiments, the total energy delivery time may be less about 30 seconds, less than about 1 minute, less than about 90 seconds, or less than about 2 minutes. As used herein, the "total energy delivery time" refers to the time period during which the waveform is supplied to the interventional element and/or catheter (including those periods of time between pulses of current).

The duty cycle of the applied electrical signal can also be selected to achieve the desired clot-adhesion characteristics without ablating tissue or promoting new clot formation. In some embodiments, the duty cycle can be between about 5% about 99% or between about 5% to about 20%. According to some embodiments, the duty cycle may be about 10%, about 20%, about 30%, about 40%, or about 50%. In yet other embodiments, a constant current may be used, in which the duty cycle is 100%. For 100% duty cycle embodiments, a lower time or current may be used to avoid delivering excess total energy to the treatment site.

Table 1 presents a range of values for power delivery parameters of different waveforms. For each of the conditions set forth in Table 1, a resistance of 1 kohm and a frequency of 1 kHz (for the Square, Triangle, and Composite conditions) was used. The Constant conditions represent a continuous and steady current applied for the duration, i.e. 100% duty cycle. The Peak Current 1 column represents the peak current for the corresponding waveform. For the Composite conditions, the Peak Current 2 column indicates the peak current of the second portion of the waveform. For example, referring back to Figure 7C, Peak Current 1 would correspond to the current at the top of the triangular portion of the waveform, while Peak Current 2 would correspond to the current at the top of the square portion of the waveform.

**Table 1**

| Condition | Peak Current 1 (mA) | Peak Current 2 (mA) | Duty Cycle 1 (%) | Duty Cycle 2 (%) | Peak Voltage (V) | Pulse Width (ms) | Total Time (s) | Total Charge (mC) | Total Energy (@ R=1000 ohm) (mJ) | Total Energy (@ R=50 ohm) (mJ) |
|---|---|---|---|---|---|---|---|---|---|---|
| Constant 1 | 2 | 0 | 100 | 0 | 2 | n/a | 120 | 240 | 480 | 24 |
| Constant 2 | 2 | 0 | 100 | 0 | 2 | n/a | 60 | 120 | 240 | 12 |
| Constant 3 | 10 | 0 | 100 | 0 | 10 | n/a | 60 | 600 | 6000 | 300 |
| Constant 4 | 20 | 0 | 100 | 0 | 20 | n/a | 60 | 1200 | 24000 | 1200 |
| Constant 5 | 10 | 0 | 100 | 0 | 10 | n/a | 120 | 1200 | 12000 | 600 |
| Constant 6 | 1 | 0 | 100 | 0 | 1 | n/a | 120 | 120 | 120 | 6 |
| Constant 7 | 0.5 | 0 | 100 | 0 | 1 | n/a | 120 | 60 | 30 | 1.5 |
| Constant 8 | 0.5 | 0 | 100 | 0 | 1 | n/a | 60 | 30 | 15 | 0.75 |
| Square 1 | 10 | 0 | 10 | 0 | 10 | 0.1 | 120 | 120 | 1200 | 60 |
| Square 2 | 4 | 0 | 50 | 0 | 4 | 0.5 | 120 | 240 | 960 | 48 |
| Square 3 | 20 | 0 | 10 | 0 | 20 | 0.1 | 120 | 240 | 4800 | 240 |
| Square 4 | 20 | 0 | 10 | 0 | 20 | 0.1 | 60 | 120 | 2400 | 120 |
| Square 5 | 10 | 0 | 10 | 0 | 10 | 0.1 | 60 | 60 | 600 | 30 |
| Triangle 1 | 10 | 0 | 10 | 0 | 10 | 0.1 | 120 | 60 | 1200 | 60 |
| Triangle 2 | 20 | 0 | 10 | 0 | 20 | 0.1 | 120 | 120 | 4800 | 240 |
| Composite 1 | 20 | 1 | 10 | 20 | 20 | 0.3 | 120 | 144 | 4824 | 264 |
| Composite 2 | 10 | 2 | 10 | 20 | 10 | 0.3 | 120 | 108 | 1296 | 156 |

As seen in Table 1, the periodic waveforms (Square, Triangle, and Composite conditions) achieve higher peak currents with lower overall charge delivered than the corresponding Constant conditions. For example, in condition Constant 4, a peak current of 20 mA corresponds to a total energy delivered of 24,000 mJ, while condition Square 3 delivers a peak current of 20 mA with a total energy of only 4,800 mJ. Conditions Triangle 2 and Composite 1 similarly deliver lower total energy while maintaining a peak current of 20 mA. Since clot-adhesion appears to be driven by peak current, these periodic waveforms can therefore offer improved clot adhesion while reducing the risk of damaging tissue at the treatment site or promoting new clot formation. Table 1 also indicates that the Triangle and Composite conditions achieve higher peak currents with lower overall charge delivered than the corresponding Square conditions. For example, condition Square 3 has a peak current of 20 mA and a total charge delivered of 240 mC, while condition Triangle 2 has a peak current of 20 mA but a total charge delivered of only 120 mC, and condition Composite 1 has a peak current of 20 mA and a total charge delivered of only 144 mC. As such, these non-square waveforms provide additional benefits by delivering desirable peak current while reducing the overall charge delivered to the treatment site.

Although Table 1 represents a series of waveforms with a single frequency (1 kHz), in some embodiments the frequency of the pulsed-DC waveforms can be controlled to achieve the desired effects. For example, in some embodiments the frequency of the waveform can be between 1 Hz and 1 MHz, between 1Hz and 1 kHz, or between 500 Hz to 1 kHz.

### V. Conclusion

This disclosure is not intended to be exhaustive or to limit the present technology to the precise forms disclosed herein. Although specific embodiments are disclosed herein for illustrative purposes, various equivalent modifications are possible without deviating from the present technology, as those of ordinary skill in the relevant art will recognize. In some cases, well-known structures and functions have not been shown and/or described in detail to avoid unnecessarily obscuring the description of the embodiments of the present technology. Although steps of methods may be presented herein in a particular order, in alternative embodiments the steps may have another suitable order. Similarly, certain aspects of the present technology disclosed in the context of particular embodiments can be combined or eliminated in other embodiments. Furthermore, while advantages associated with certain embodiments may have been disclosed in the context of those embodiments, other embodiments can also exhibit such advantages, and not all embodiments need necessarily exhibit such advantages or other advantages disclosed herein to fall within the scope of the present technology. Accordingly, this disclosure and associated technology can encompass other embodiments not expressly shown and/or described herein.

Throughout this disclosure, the singular terms "a," "an," and "the" include plural referents unless the context clearly indicates otherwise. Similarly, unless the word "or" is expressly limited to mean only a single item exclusive from the other items in reference to a list of two or more items, then the use of "or" in such a list is to be interpreted as including (a) any single item in the list, (b) all of the items in the list, or (c) any combination of the items in the list. Additionally, the terms "comprising" and the like are used throughout this disclosure to mean including at least the recited feature(s) such that any greater number of the same feature(s) and/or one or more additional types of features are not precluded. Directional terms, such as "upper," "lower," "front," "back," "vertical," and "horizontal," may be used herein to express and clarify the relationship between various elements. It should be understood that such terms do not denote absolute orientation. Reference herein to "one embodiment," "an embodiment," or similar formulations means that a particular feature, structure, operation, or characteristic described in connection with the embodiment can be included in at least one embodiment of the present technology. Thus, the appearances of such phrases or formulations herein are not necessarily all referring to the same embodiment. Furthermore, various particular features, structures, operations, or characteristics may be combined in any suitable manner in one or more embodiments. The scope of the invention is defined by the claims.

## Claims

1. A thrombectomy device comprising:
an interventional element (100,600,700,800) configured to be advanced intravascularly to a treatment site in a corporeal lumen and to engage a thrombus therein, wherein the interventional element (100,600,700,800) possesses an inner conductive material and an overlying material that has a lower electrical conductivity than the inner conductive material, **characterized by**
the overlying material having a thickness gradient that decreases from a first thickness in a proximal portion of the interventional element (100,600,700,800) to a second non-zero thickness in a distal portion of the interventional element (100,600,700,800) such that a surface electrical conductivity of the interventional element (100,600,700,800) has a gradient that increases from the proximal portion of the interventional element to the distal portion of the interventional element (100,600,700,800), wherein the surface electrical conductivity is greater than zero in the proximal portion.

2. The thrombectomy device of Claim 1, wherein the interventional element (100,600,700,800) comprises interconnected struts (101) formed of the inner conductive material, with the overlying material positioned over the inner conductive material, optionally wherein the interventional element (100,600,700,800) comprises openings formed between the struts (101).

3. The thrombectomy device of Claim 1, wherein the interventional element (100,600,700,800) comprises a mesh formed of the inner conductive material, with the overlying material positioned over the inner conductive material.

4. The thrombectomy device of Claim 3, wherein the mesh is in a generally tubular or cylindrical configuration.

5. The thrombectomy device of Claim 1, wherein the interventional element (100,600,700,800) comprises a plurality of conductive regions (R1-R7) arranged in a series along the length of the interventional element (100,600,700,800).

6. The thrombectomy device of Claim 5, wherein each conductive region in the series abuts at least one other conductive region in the series at a proximal or distal end of said each conductive region.

7. The thrombectomy device of Claim 5, wherein the series comprises a first conductive region of the plurality, which abuts a second conductive region of the plurality, at a proximal or distal end of the first conductive region.

8. The thrombectomy device of Claim 5, wherein the series has N regions and the surface conductivity SC of any given region Rx can be related to the surface conductivity SC of a proximally-adjacent region Rx-1 by the relation [*SC_{Rx}* > *SC*_{*Rx*-1}] where x is a positive integer ranging from 2 to N, optionally wherein SC_{R1} is no less than zero.

9. The thrombectomy device of Claim 5, wherein the surface conductivity of the interventional element (100,600,700,800) increases from one region to the next along the series, proceeding distally.

10. The thrombectomy device of Claim 5, wherein, within each region in the series, a uniform thickness of the overlying material is present, and the thickness of the overlying material decreases from one region to the next along the series.

11. The thrombectomy device of Claim 6, wherein each region in the series comprises the entirety of the interventional element (100,600,700,800) from a first location along the length of the interventional element (100,600,700,800) to a second, distal location along the length of the interventional element (100,600,700,800).

12. The thrombectomy device of Claim 5, wherein the series comprises three or more regions.

13. The thrombectomy device of Claim 1, wherein the inner conductive material is metal, and wherein the overlying material is a metal oxide.

14. The thrombectomy device of Claim 13, wherein the metal comprises Nitinol, and
wherein the overlying material is Ti-Ni-O oxide, optionally wherein the metal oxide has a thickness between about 0 to about 200 nm (0 to about 2000 Angstroms).

15. The thrombectomy system of Claim 1, wherein the overlying material comprises a surface treatment, optionally wherein the surface treatment comprises electrochemical anodization.

16. The thrombectomy device of Claim 1, wherein the interventional element (100,600,700,800) comprises a stent or stent retriever.

17. The thrombectomy device of Claim 1, further comprising an elongate manipulation member coupled to the interventional element (100,600,700,800), optionally wherein the manipulation member is configured to facilitate advancement of the interventional element (100,600,700,800) within a blood vessel of a patient, and/or wherein the manipulation member comprises an electrical conductor which is electrically coupled to the interventional element (100,600,700,800).

18. The thrombectomy device of Claim 1, further comprising one or more radiopaque markers (220) along the interventional element (100,600,700,800).

19. A method of treating a thrombectomy device, comprising:
providing an interventional element (100,600,700,800) configured to engage a thrombus within a blood vessel and to deliver electrical current thereto, the interventional element (100,600,700,800) comprising an electrically conductive first material; and
surface treating the interventional element (100,600,700,800) to form a second material over the first material, the second material having a lower electrical conductivity than the first material, **characterized in that**
the second material has a thickness gradient that
decreases from a first thickness in a proximal portion of the interventional element (100,600,700,800) to a second non-zero thickness in a distal portion of the interventional element (100,600,700,800) such that a surface electrical conductivity of the interventional element (100,600,700,800) has a gradient that increases from the proximal portion of the interventional element (100,600,700,800) to the distal portion of the interventional element.

20. The method of Claim 19, wherein the first material is metallic, and second material comprises a metal oxide and/or wherein the surface treating comprises immersing a distal portion of the interventional element (100,600,700,800) in an electrolyte for a first period of time and immersing a proximal portion of the interventional element (100,600,700,800) in the electrolyte for a second period of time greater than the first period of time.

## Patentansprüche

1. Thrombektomievorrichtung, umfassend:
ein interventionelles Element (100,600,700,800), das konfiguriert ist, um zu einer Behandlungsstelle in einem Körperlumen intravaskulär vorgeschoben zu werden und darin einen Thrombus in Eingriff zu nehmen, wobei das interventionelle Element (100,600,700,800) ein inneres leitfähiges Material und ein darüberliegendes Material besitzt, das eine geringere elektrische Leitfähigkeit als das innere leitfähige Material aufweist, **gekennzeichnet durch** das darüberliegende Material, das einen Dickengradienten aufweist, der von einer ersten Dicke in einem proximalen Abschnitt des interventionellen Elements (100,600,700,800) zu einer zweiten Dicke ungleich Null in einem distalen Abschnitt des interventionellen Elements (100,600,700,800) derart abnimmt, dass eine elektrische Oberflächenleitfähigkeit des interventionellen Elements (100,600,700,800) einen Gradienten aufweist, der von dem proximalen Abschnitt des interventionellen Elements zu dem distalen Abschnitt des interventionellen Elements (100,600,700,800) zunimmt, wobei die elektrische Oberflächenleitfähigkeit in dem proximalen Abschnitt größer als Null ist.

2. Thrombektomievorrichtung nach Anspruch 1, wobei das interventionelle Element (100,600,700,800) miteinander verbundene Streben (101) umfasst, die aus dem inneren leitfähigen Material ausgebildet sind, wobei das darüberliegende Material über dem inneren leitfähigen Material positioniert ist, optional wobei das interventionelle Element (100,600,700,800) Öffnungen umfasst, die zwischen den Streben (101) ausgebildet sind.

3. Thrombektomievorrichtung nach Anspruch 1, wobei das interventionelle Element (100,600,700,800) ein Geflecht umfasst, das aus dem inneren leitfähigen Material ausgebildet wird, wobei das darüberliegende Material über dem inneren leitfähigen Material positioniert ist.

4. Thrombektomievorrichtung nach Anspruch 3, wobei das Geflecht eine im Wesentlichen rohrförmige oder zylindrische Konfiguration ist.

5. Thrombektomievorrichtung nach Anspruch 1, wobei das interventionelle Element (100,600,700,800) eine Vielzahl von leitfähigen Regionen (R1-R7) umfasst, die in einer Reihe entlang der Länge des interventionellen Elements (100,600,700,800) angeordnet ist.

6. Thrombektomievorrichtung nach Anspruch 5, wobei jede leitfähige Region in der Reihe an einem proximalen oder distalen Ende jeder leitfähigen Region an mindestens eine andere leitfähige Region in der Reihe angrenzt.

7. Thrombektomievorrichtung nach Anspruch 5, wobei die Reihe eine erste leitfähige Region der Vielzahl umfasst, die an einem proximalen oder distalen Ende der ersten leitfähigen Region an eine zweite leitfähige Region der Vielzahl angrenzt.

8. Thrombektomievorrichtung nach Anspruch 5, wobei die Reihe N Regionen aufweist und die Oberflächenleitfähigkeit SC einer beliebigen Region Rx auf die Oberflächenleitfähigkeit SC einer proximal angrenzenden Region Rx-1 durch die Beziehung *[SC_{Rx} > SC_{Rx-1}]* bezogen werden kann, wobei x eine positive ganze Zahl in einem Bereich von 2 bis N ist, optional wobei SC_{R1} nicht kleiner als Null ist.

9. Thrombektomievorrichtung nach Anspruch 5, wobei die Oberflächenleitfähigkeit des interventionellen Elements (100,600,700,800) entlang der Reihe, distal voranschreitend, von einer Region zu der nächsten zunimmt.

10. Thrombektomievorrichtung nach Anspruch 5, wobei innerhalb jeder Region in der Reihe eine gleichmäßige Dicke des darüberliegenden Materials vorhanden ist und die Dicke des darüberliegenden Materials entlang der Reihe von einer Region zu der nächsten abnimmt.

11. Thrombektomievorrichtung nach Anspruch 6, wobei jede Region in der Reihe die Gesamtheit des interventionellen Elements (100,600,700,800) von einem ersten Ort entlang der Länge des interventionellen Elements (100,600,700,800) zu einem zweiten, distalen Ort entlang der Länge des interventionellen Elements (100,600,700,800) umfasst.

12. Thrombektomievorrichtung nach Anspruch 5, wobei die Reihe drei oder mehr Regionen umfasst.

13. Thrombektomievorrichtung nach Anspruch 1, wobei das innere leitfähige Material Metall ist und wobei das darüberliegende Material ein Metalloxid ist.

14. Thrombektomievorrichtung nach Anspruch 13, wobei das Metall Nitinol umfasst und wobei das darüberliegende Material Ti-Ni-O-Oxid ist, optional wobei das Metalloxid eine Dicke zwischen etwa 0 und etwa 200 nm (0 bis etwa 2000 Angström) aufweist.

15. Thrombektomievorrichtung nach Anspruch 1, wobei das darüberliegende Material eine Oberflächenbehandlung umfasst, optional wobei die Oberflächenbehandlung eine elektrochemische Anodisierung umfasst.

16. Thrombektomievorrichtung nach Anspruch 1, wobei das interventionelle Element (100,600,700,800) einen Stent oder einen Stent-Retriever umfasst.

17. Thrombektomievorrichtung nach Anspruch 1, ferner umfassend ein längliches Manipulationselement, das mit dem interventionellen Element (100,600,700,800) gekoppelt ist, optional wobei das Manipulationselement konfiguriert ist, um ein Vorschieben des interventionellen Elements (100,600,700,800) innerhalb eines Blutgefäßes eines Patienten zu erleichtern, und/oder wobei das Manipulationselement einen elektrischen Leiter umfasst, der mit dem interventionellen Element (100,600,700,800) elektrisch gekoppelt ist.

18. Thrombektomievorrichtung nach Anspruch 1, ferner umfassend einen oder mehrere röntgendichte Marker (220) entlang des interventionellen Elements (100,600,700,800).

19. Verfahren zum Behandeln einer Thrombektomievorrichtung, umfassend:
Bereitstellen eines interventionellen Elements (100,600,700,800), das konfiguriert ist, um einen Thrombus innerhalb eines Blutgefäßes in Eingriff zu nehmen und elektrischen Strom dorthin abzugeben, das interventionelle Element (100,600,700,800) umfassend ein elektrisch leitfähiges erstes Material; und
Oberflächenbehandeln des interventionellen Elements (100,600,700,800), um ein zweites Material über dem ersten Material auszubilden, wobei das zweite Material eine geringere elektrische Leitfähigkeit als das erste Material aufweist, **dadurch gekennzeichnet, dass** das zweite Material einen Dickengradienten aufweist, der von einer ersten Dicke in einem proximalen Abschnitt des interventionellen Elements (100,600,700,800) zu einer zweiten Dicke ungleich Null in einem distalen Abschnitt des interventionellen Elements (100,600,700,800) derart abnimmt, dass eine elektrische Oberflächenleitfähigkeit des interventionellen Elements (100,600,700,800) einen Gradienten aufweist, der von dem proximalen Abschnitt des interventionellen Elements (100,600,700,800) zu dem distalen Abschnitt des interventionellen Elements zunimmt.

20. Verfahren nach Anspruch 19, wobei das erste Material metallisch ist und das zweite Material ein Metalloxid umfasst und/oder wobei die Oberflächenbehandlung ein Eintauchen eines distalen Abschnitts des interventionellen Elements (100,600,700,800) in einen Elektrolyten für einen ersten Zeitraum und das Eintauchen eines proximalen Abschnitts des interventionellen Elements (100,600,700,800) in den Elektrolyten für einen zweiten Zeitraum umfasst, der länger als der erste Zeitraum ist.

## Revendications

1. Dispositif de thrombectomie comprenant :
un élément d'intervention (100, 600, 700, 800) configuré pour être avancé par voie intravasculaire vers un site de traitement dans une lumière corporelle et pour engager un thrombus à l'intérieur de celle-ci, dans lequel l'élément d'intervention (100, 600, 700, 800) possède un matériau conducteur interne et un matériau recouvrant qui a une conductivité électrique plus faible que le matériau conducteur interne, **caractérisé par le** matériau recouvrant ayant un gradient d'épaisseur qui diminue d'une première épaisseur dans une partie proximale de l'élément d'intervention (100, 600, 700, 800) à une seconde épaisseur non nulle dans une partie distale de l'élément d'intervention (100, 600, 700, 800) de telle sorte qu'une conductivité électrique de surface de l'élément d'intervention (100, 600, 700, 800) présente un gradient qui augmente de la partie proximale de l'élément d'intervention à la partie distale de l'élément d'intervention (100, 600, 700, 800), dans lequel la conductivité électrique de surface est supérieure à zéro dans la partie proximale.

2. Dispositif de thrombectomie selon la revendication 1, dans lequel l'élément d'intervention (100, 600, 700, 800) comprend des entretoises interconnectées (101) formées du matériau conducteur interne, avec le matériau de recouvrement positionné sur le matériau conducteur interne, éventuellement dans lequel l'élément d'intervention (100, 600, 700, 800) comprend des ouvertures formées entre les entretoises (101).

3. Dispositif de thrombectomie selon la revendication 1, dans lequel l'élément d'intervention (100, 600, 700, 800) comprend une maille formée du matériau conducteur interne, le matériau de recouvrement étant positionné sur le matériau conducteur interne.

4. Dispositif de thrombectomie selon la revendication 3, dans lequel la maille a une configuration généralement tubulaire ou cylindrique.

5. Dispositif de thrombectomie selon la revendication 1, dans lequel l'élément d'intervention (100, 600, 700, 800) comprend une pluralité de régions conductrices (R1-R7) agencées en série le long de la longueur de l'élément d'intervention (100, 600, 700, 800).

6. Dispositif de thrombectomie selon la revendication 5, dans lequel chaque région conductrice de la série est en contact avec au moins une autre région conductrice de la série au niveau d'une extrémité proximale ou distale de chaque dite région conductrice.

7. Dispositif de thrombectomie selon la revendication 5, dans lequel la série comprend une première région conductrice de la pluralité, qui est en contact avec une seconde région conductrice de la pluralité, au niveau d'une extrémité proximale ou distale de la première région conductrice.

8. Dispositif de thrombectomie selon la revendication 5, dans lequel la série comporte N régions et la conductivité de surface SC d'une région donnée Rx peut être reliée à la conductivité de surface SC d'une région proximalement adjacente Rx-1 par la relation *[SC_{Rx} > SC_{Rx-1}]* où x est un nombre entier positif allant de 2 à N, éventuellement dans lequel SC_{R1} n'est pas inférieur à zéro.

9. Dispositif de thrombectomie selon la revendication 5, dans lequel la conductivité de la surface de l'élément d'intervention (100, 600, 700, 800) augmente d'une région à l'autre le long de la série, en procédant de manière distale.

10. Dispositif de thrombectomie selon la revendication 5, dans lequel, à l'intérieur de chaque région de la série, une épaisseur uniforme du matériau de recouvrement est présente, et l'épaisseur du matériau de recouvrement diminue d'une région à l'autre le long de la série.

11. Dispositif de thrombectomie selon la revendication 6, dans lequel chaque région de la série comprend la totalité de l'élément d'intervention (100, 600, 700, 800) à partir d'un premier emplacement le long de la longueur de l'élément d'intervention (100 600 700 800) jusqu'à un second emplacement distal sur la longueur de l'élément d'intervention (100, 600, 700, 800).

12. Dispositif de thrombectomie selon la revendication 5, dans lequel la série comprend trois régions ou plus.

13. Dispositif de thrombectomie selon la revendication 1, dans lequel le matériau conducteur interne est un métal, et dans lequel le matériau de recouvrement est un oxyde métallique.

14. Dispositif de thrombectomie selon la revendication 13, dans lequel le métal comprend du Nitinol, et dans lequel le matériau de recouvrement est de l'oxyde Ti-Ni-O, éventuellement dans lequel l'oxyde métallique a une épaisseur comprise entre environ 0 et environ 200 nm (0 et environ 2 000 Angstroms).

15. Système de thrombectomie selon la revendication 1, dans lequel le matériau de recouvrement comprend un traitement de surface, éventuellement dans lequel le traitement de surface comprend une anodisation électrochimique.

16. Dispositif de thrombectomie selon la revendication 1, dans lequel l'élément interventionnel (100, 600, 700, 800) comprend une endoprothèse ou un récupérateur d'endoprothèse.

17. Dispositif de thrombectomie selon la revendication 1, comprenant en outre un élément de manipulation allongé couplé à l'élément d'intervention (100, 600, 700, 800), éventuellement dans lequel l'élément de manipulation est configuré pour faciliter l'avancement de l'élément d'intervention (100, 600, 700, 800) dans un vaisseau sanguin d'un patient, et/ou dans lequel l'élément de manipulation comprend un conducteur électrique qui est couplé électriquement à l'élément d'intervention (100, 600, 700, 800).

18. Dispositif de thrombectomie selon la revendication 1, comprenant en outre un ou plusieurs marqueurs radio-opaques (220) le long de l'élément d'intervention (100, 600, 700, 800).

19. Procédé de traitement d'un dispositif de thrombectomie, comprenant :
la fourniture d'un élément d'intervention (100, 600, 700, 800) configuré pour engager un thrombus à l'intérieur d'un vaisseau sanguin et pour y fournir un courant électrique, l'élément d'intervention (100, 600, 700, 800) comprenant un premier matériau conducteur d'électricité ; et
le traitement de la surface de l'élément d'intervention (100, 600, 700, 800) pour former un second matériau sur le premier matériau, le second matériau ayant une conductivité électrique inférieure à celle du premier matériau,
**caractérisé en ce que** le second matériau a un gradient d'épaisseur qui diminue d'une première épaisseur dans une partie proximale de l'élément d'intervention (100, 600, 700, 800) à une seconde épaisseur non nulle dans une partie distale de l'élément d'intervention (100, 600, 700, 800) de telle sorte qu'une conductivité électrique de surface de l'élément d'intervention (100, 600, 700, 800) présente un gradient qui augmente de la partie proximale de l'élément d'intervention (100, 600, 700, 800) à la partie distale de l'élément d'intervention.

20. Procédé selon la revendication 19, dans lequel le premier matériau est métallique et le second matériau comprend un oxyde métallique et/ou dans lequel le traitement de surface comprend l'immersion d'une partie distale de l'élément d'intervention (100, 600, 700, 800) dans un électrolyte pendant un premier laps de temps et l'immersion d'une partie proximale de l'élément d'intervention (100, 600, 700, 800) dans l'électrolyte pour un second laps de temps plus long que le premier laps de temps.
